(19) Europäisches Patentamt
European Patent Office
Office européen des brevets

(11) EP 4 011 880 A1

(12) # EUROPEAN PATENT APPLICATION
published in accordance with Art. 153(4) EPC

(43) Date of publication:
**15.06.2022 Bulletin 2022/24**

(21) Application number: **20849907.9**

(22) Date of filing: **05.08.2020**

(51) International Patent Classification (IPC):
***C07D 471/04*** (2006.01) ***A61P 19/08*** (2006.01)
***A61P 19/02*** (2006.01) ***A61P 17/06*** (2006.01)
***A61P 11/00*** (2006.01) ***A61P 35/00*** (2006.01)
***A61P 35/02*** (2006.01) ***A61P 19/00*** (2006.01)
***A61P 37/06*** (2006.01) ***A61P 37/08*** (2006.01)
***A61K 31/437*** (2006.01) ***A61K 31/4545*** (2006.01)
***A61K 31/54*** (2006.01)

(52) Cooperative Patent Classification (CPC):
**A61K 31/437; A61K 31/4545; A61K 31/54; A61P 1/00; A61P 11/00; A61P 17/06; A61P 19/00; A61P 19/02; A61P 19/08; A61P 35/00; A61P 35/02; A61P 37/06; A61P 37/08; C07D 471/04**

(86) International application number:
**PCT/CN2020/107028**

(87) International publication number:
**WO 2021/023207 (11.02.2021 Gazette 2021/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.08.2019 CN 201910721525**

(71) Applicant: **Jiangsu Carephar Pharmaceutical Co., Ltd**
**Jiangsu 210042 (CN)**

(72) Inventors:
- **QIN, Yinlin**
**Nanjing, Jiangsu 210042 (CN)**
- **SU, Mei**
**Nanjing, Jiangsu 210042 (CN)**

(74) Representative: **karo IP**
**karo IP Patentanwälte**
**Kahlhöfer Rößler Kreuels PartG mbB**
**Postfach 32 01 02**
**40416 Düsseldorf (DE)**

(54) **JAK KINASE INHIBITOR AND USE THEREOF**

(57) The present invention discloses a JAK kinase inhibitor and use thereof. The JAK kinase inhibitor is a compound represented by formula (I) or a stereoisomer or tautomer thereof or a pharmaceutically acceptable salt thereof or a solvate or prodrug thereof. The present invention further provides the use of the compound of formula I of the present invention in preparation of drugs for preventing or treating JAK kinase-related diseases, especially in preparation of drugs for preventing and/or treating diseases involving cartilage degradation and bone and/or joint degradation, conditions involving inflammation or immune response, endotoxin-driven disease states, cancer, and organ transplantation rejection.

EP 4 011 880 A1

## Description

## TECHNICAL FIELD

The present invention belongs to the technical field of medicines and specifically relates to a novel nitrogen-containing five-membered heterocyclic pyridine compound, a preparation method thereof and a pharmaceutical composition containing the same, and use thereof for modulating Janus kinase (JAK) activity and for the treatment and/or prevention of diseases associated with JAK activity.

## BACKGROUND

Chondrocytes are the major cellular components of avascular tissue. In normal articular cartilage, the chondrocytes account for approximately 5% of the tissue volume, while the extracellular matrix accounts for the remaining 95% of the tissue. The chondrocytes secrete matrix components, mainly proteoglycans and collagen, which in turn provide the chondrocytes with an environment suitable for their survival under mechanical stress. In cartilage, collagen type II and collagen type IX form a fibrillar structure that provides great mechanical strength to the cartilage. Proteoglycans can absorb water and are responsible for the elastic and shock-absorbing properties of cartilage. One of the functional roles of cartilage is to provide the bony parts with a smooth joint connection to each other. Therefore, the loss of articular cartilage causes the bones to rub against each other, resulting in pain and loss of motion. In inflammatory arthritis such as rheumatoid arthritis, cartilage degeneration is caused by proteases (collagenases) secreted by inflamed tissue (e.g., inflamed synovium). Chondrocytes in damaged cartilage often show reduced chondrogenic activity and/or increased cartilage degenerative activity, and cartilage degeneration is the main disease marker for the development of rheumatoid arthritis and osteoarthritis. Rheumatoid arthritis (RA) is a chronic degenerative joint disease characterized by inflammation and destruction of joint structures, which may lead to functional derangement of the joints, causing substantial incapacity and pain, and even premature death. Therefore, the aim of RA treatment is not only to delay the disease, but also to reduce the pain and protect the joint destruction.

JAK kinases belong to the Janus kinase (JAK) family of cytoplasmic tyrosine kinases, which are involved in cytokine receptor-mediated intracellular signaling. The JAK kinase family includes four members: JAK1, JAK2, JAK3 and TYK2. JAK recruits cytokine receptors, binds cytokines and subsequently dimerizes the cytokine receptors and shared receptor subunits. JAK is then activated by autophosphorylation and/or transphosphorylation of another JAK, leading to receptor phosphorylation as well as recruitment and phosphorylation of members of signal transducer and activator of transcription (STAT). Phosphorylated SATA is dimerized and translocated to the nucleus where they bind to the enhancer regions of cytokine response genes. JAK plays an important role in regulating the biological response functions of various cytokine receptor families. JAK1 knockout mice have an early postnatal lethal factor phenotype and the nervous system is damaged, resulting in congenital defects in the young mice. It has been shown that JAK1 knockout mice may develop defective secretion of thymocytes and B cells, and JAK1 knockout tissues have a significantly diminished response to IL-6 and IL-10.

The association of JAK with autoimmune diseases has been clinically established, with mutations in JAK as well as in the upstream signaling components r-c receptor chain and IL7 receptor detected in approximately 70% of severe combined immunodeficiency cases. Therefore, targeting the JAK family could provide new therapeutic drugs in the immune-inflammation field.

Clinical conditions in which JAK family member JAK2 is involved include myeloproliferative disorders, cancer especially leukemia such as acute myeloid leukemia, acute lymphoblastic leukemia or solid tumors such as uterine leiomyosarcoma, prostate cancer and other related diseases. Therefore, drugs targeting the JAK family may also offer new options for the treatment of the above diseases.

## SUMMARY

The present invention provides novel JAK kinase inhibitors, preparation methods thereof, pharmaceutical compositions containing these kinase inhibitors and use thereof.

More specifically, the present invention relates to a compound with a structure shown in formula (I) or a stereoisomer or tautomer thereof or a pharmaceutically acceptable salt thereof or a solvate or prodrug thereof:

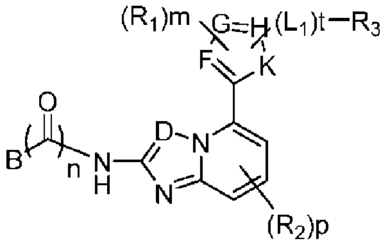

Formula I

wherein:

B is independently selected from substituted or unsubstituted $C_3$-$C_6$ cycloalkyl, aryl, and 5- to 6-membered heteroaryl ring having 1 to 2 ring heteroatoms independently selected from N, O and S, wherein the 5- to 6-membered heteroaryl ring is optionally substituted with one or more substituents independently selected from halogen, fluorinated or unfluorinated $C_1$-$C_6$ alkyl, fluorinated or unfluorinated $C_1$-$C_6$ alkoxy, and fluorinated or unfluorinated $C_1$-$C_6$ alkylamino;
D is independently selected from C and N;
F, G, H, and K are independently selected from C, N, S, and O;
$L_1$ is absent or is independently selected from single bond, $-CH_2-$, $-(CH_2)_xO(CH_2)_y-$, $-(CH_2)_xNH(CH_2)_y-$, $-CH_2O-$, -C(O)-, $-CON(R_4)-$, $-CH_2N(R_4)-$, $-CONH(CH_2)_y-$, $-N(R_4)-$, $-SO_2N(R_4)-$, $-S(O)_2-$, and -N(Me)-;
$R_4$ is independently selected from H, $C_1$-$C_6$ alkyl, substituted $C_1$-$C_6$ alkyl, $C_1$-$C_3$ ether $C_1$-$C_3$ alkyl, and methylsulfonyl;
$R_1$ is independently selected from H, $-NH_2$, -COOH, substituted or unsubstituted $C_1$-$C_6$ alkyl, acyl, substituted or unsubstituted acylamino, substituted or unsubstituted $C_1$-$C_6$ alkoxy, halogen, hydroxyl, substituted or unsubstituted $C_1$-$C_3$ ester group, and substituted or unsubstituted heteroaryl;
$R_2$ is absent or is independently selected from H, F, Cl, and Me;
$R_3$ is independently selected from H, substituted or unsubstituted sulfonyl, substituted or unsubstituted sulfonamido, substituted or unsubstituted 5- to 6-membered heterocyclic ring having at least one heteroatom and optionally having a second ring heteroatom independently selected from N and S, substituted or unsubstituted $C_1$-$C_6$ alkane, and a substituent is independently selected from 5- to 6-membered heteroaryl and methoxy, substituted or unsubstituted $C_3$-$C_7$ cycloalkyl, substituted or unsubstituted 4- to 7-membered heterocycloalkyl, and substituted or unsubstituted 5- to 7-membered heteroaryl; and
m is 0, 1, 2, or 3; n is 0, 1, 2, or 3; p is 0, 1, or 2; and t is 0, 1, 2, or 3.

In some embodiments, F, G, H, and K are not C at the same time.

In one embodiment, B is selected from the following substituted or unsubstituted groups: cyclopropane, cyclobutane, pyrazolyl, pyridyl, and imidazolyl; and a substituent is selected from F, Cl, Br, methyl, ethyl, propyl, isopropyl, and trifluoromethyl.

In a specific embodiment, B is substituted or unsubstituted cyclopropane; and the substituent is selected from F, Cl, Br, methyl, ethyl, propyl, isopropyl, and trifluoromethyl.

In one embodiment, D is C or N.

In a specific embodiment, D is N.

In one embodiment, n is 1.

In one embodiment, K is S, and F, G, and H are C.

In one embodiment, $R_1$ is independently selected from H, $-NH_2$, -COOH, hydroxyl, halogen, substituted or unsubstituted $C_1$-$C_3$ alkyl, substituted or unsubstituted $C_1$-$C_3$ acyl, substituted or unsubstituted $C_1$-$C_3$ acylamino, substituted or unsubstituted $C_1$-$C_3$ alkoxy, and substituted or unsubstituted $C_1$-$C_3$ ester group; in a preferred embodiment, $R_1$ is independently selected from H, $-NH_2$, -COOH, hydroxyl, halogen, methyl, ethyl, propyl, isopropyl, formylamino and carbomethoxy.

In one embodiment, m is 0 or 1, and more preferably, m is 0.

In one embodiment, $R_2$ is absent or is H.

In a specific embodiment, $R_2$ is absent.

In one embodiment, P is 0.

In one embodiment, $L_1$ is absent or independently selected from single bond, $-CH_2-$, $-(CH_2)_xO(CH_2)_y-$, $-(CH_2)_xNH(CH_2)_y-$, $-CONH(CH_2)_y-$, and $-N(R_4)-$; $R_4$ is independently selected from H, methyl, ethyl, propyl, ethyl methyl ether, methyl methyl ether, ethyl ethyl ether; and x or y is independently selected from 0, 1 or 2. In one more preferred embodiment, $L_1$ is absent or is independently selected from single bond, $-CH_2-$, $-(CH_2)_xO(CH_2)_y-$, $-(CH_2)_xNH(CH_2)_y-$,

-CONH($CH_2$)$_y$-, and -N($R_4$)-. In some more specific embodiments, $L_1$ is selected from -$CH_2$-, -($CH_2$)$_x$O($CH_2$)$_y$-, and -CONH($CH_2$)$_y$-, $R_4$ is independently selected from H, methyl, and ethyl methyl ether; and x or y is independently selected from 0, 1 or 2. In some specific embodiments, x or y is independently selected from 1 or 2.

In one embodiment, t is 1.

In one embodiment, $R_3$ is independently selected from H and the following substituted or unsubstituted groups: sulfonyl, sulfonamido, thiomorpholine 1,1-dioxide, piperidine, pyrazole, thiazole, imidazole, 1,3,4-thiadiazole, piperazine, morpholine, thiophene, oxazole, 1,3,4-oxadiazole, furan, pyrrole, 3-pyrroline, 2-pyrazoline, 1,2,3-azole, 1,2,3-triazole, 1,2,4-triazole, and pyran; and a substituent is selected from H, halogen, $C_1$-$C_3$ alkyl, $C_1$-$C_3$ haloalkyl, hydroxy $C_1$-$C_3$ alkyl, $C_3$-$C_6$ cycloalkyl, ethyl methyl ether, methyl methyl ether, and ethyl ethyl ether; in some embodiments, the substituent may also be selected from $C_1$-$C_3$ haloalkoxy; preferably, $R_3$ is independently selected from H and the following substituted or unsubstituted groups: sulfonyl, sulfonamido, thiomorpholine 1,1-dioxide, piperidine, pyrazole, thiazole, imidazole, 1,3,4-thiadiazole, piperazine, morpholine, pyrazine, thiophene, oxazole, 1,3,4-oxadiazole, furan, pyrrole, 3-pyrroline, 2-pyrazoline, 1,2,3-azole, 1,2,3-triazole, 1,2,4-triazole, and pyran; and a substituent is selected from H, halogen, methyl, ethyl, propyl, isopropyl, trifluoromethyl, hydroxyethyl, hydroxymethyl, cyclopropane, cyclobutane, ethyl methyl ether, methyl methyl ether, and ethyl ethyl ether; and in some embodiments, the substituent may also be selected from trifluoromethoxy.

The present invention also provides a compound represented by the following structure, or a stereoisomer or tautomer thereof, or a pharmaceutically acceptable salt thereof, or a solvate or prodrug thereof:

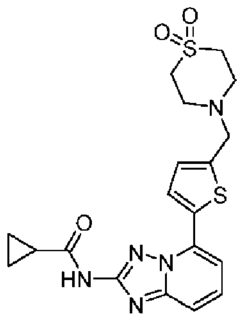

**EXP-1**

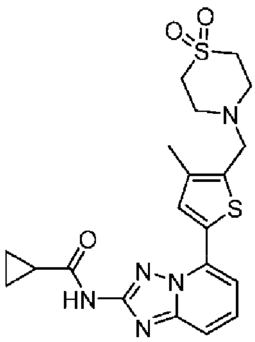

EXP-2

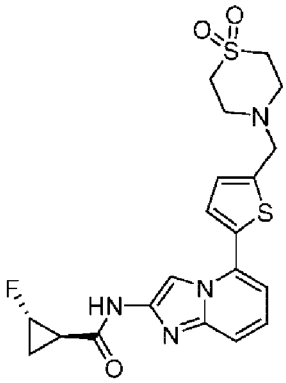

EXP-3

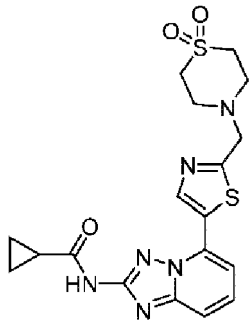

**EXP-4**

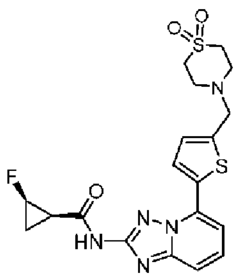

**EXP-5**

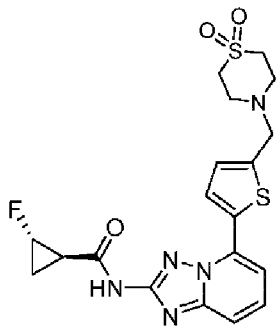

EXP-6

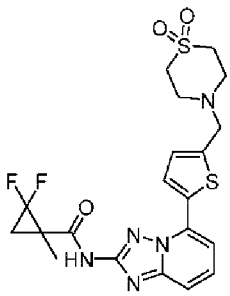

EXP-7

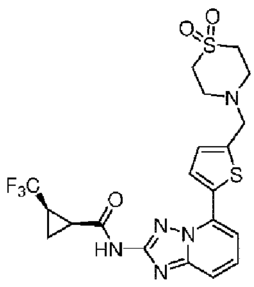

EXP-8

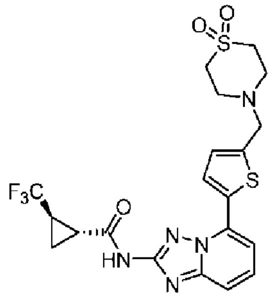

EXP-9

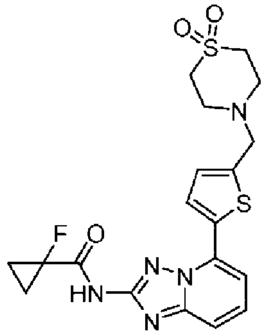

EXP-10

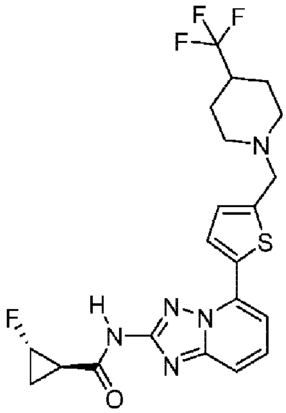

EXP-11

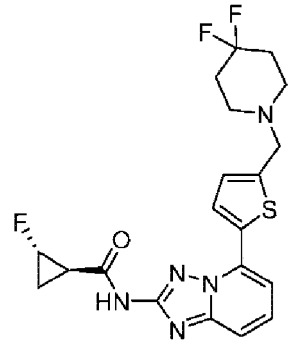

EXP-12

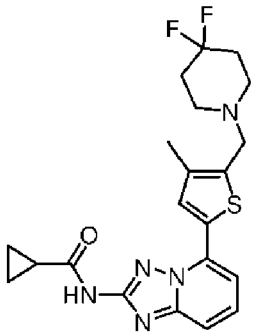

EXP-13

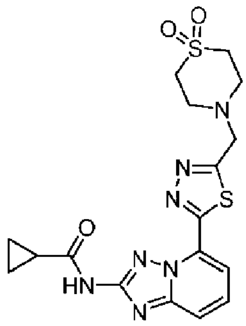

**EXP-14**

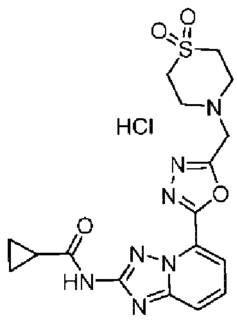

**EXP-15**

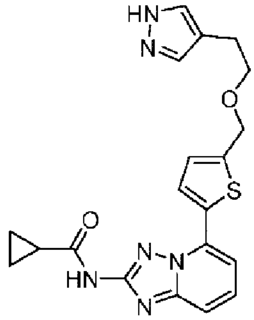

**EXP-16**

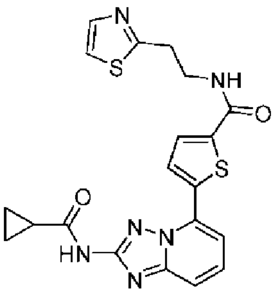

**EXP-17**

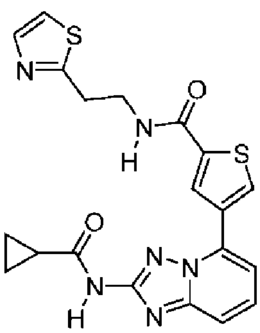

EXP-18

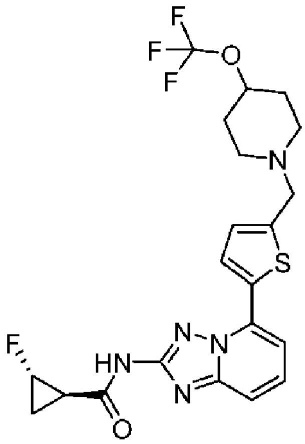

**EXP-19**

The present invention also provides a method for synthesizing the compound according to the present invention, as well as representative synthetic schemes and routes as disclosed.

The present invention also provides a pharmaceutical composition containing the compound of formula I according to the present invention or a pharmaceutically acceptable salt thereof, and a pharmaceutically acceptable diluent or carrier.

The present invention also provides use of the compound of formula I according to the present invention in preparation of drugs for preventing or treating JAK kinase-related diseases, especially in preparation of drugs for preventing and/or treating diseases involving cartilage degeneration and bone and/or joint degeneration, conditions involving inflammation or immune response, endotoxin-driven disease states, cancer, and organ transplantation rejection.

The present invention also provides a method for preventing and/or treating diseases involving cartilage degradation and bone and/or joint degradation, conditions involving inflammation or immune response, endotoxin-driven disease states, cancer and organ transplantation rejection by administering the compound according to the present invention.

In one embodiment, examples of diseases involving cartilage degradation and bone and/or joint degradation, conditions involving inflammation or immune response, endotoxin-driven disease states, cancer, and organ transplantation rejection according to the present invention include but not limited to: osteoarthritis, Crohn's disease, rheumatoid arthritis, psoriasis, allergic airway disease (such as asthma, rhinitis), juvenile idiopathic arthritis, colitis, inflammatory bowel disease, endotoxin-driven disease state, diseases with cartilage renewal damage (such as a disease with anabolic excitation of chondrocytes), congenital cartilage deformity, organ transplantation rejection, cartilage degeneration, joint degeneration, myeloproliferative disorder, leukemia (acute myeloid leukemia, acute lymphoblastic leukemia), and solid tumor (uterine leiomyosarcoma, prostate cancer), and the like.

**Definition**

The term "amino" used in the present application refers to a functional group having 1 nitrogen atom and 1 to 2 hydrogen atoms. "Amino" is generally used herein to describe primary amine, secondary amine, or tertiary amine and those skilled in the art can easily determine the amino based on the context in which the term is used in this disclosure. The term "amine" or "amine group" or "amino group" refers to a functional group containing a nitrogen atom derived from ammonia ($NH_3$). The amine group is preferably primary amine, which means that nitrogen is bonded to two hydrogen atoms and one substituent containing a substituted or unsubstituted alkyl or aryl or aliphatic or aromatic groups. The amine group may be secondary amine, which means that nitrogen is bonded to one hydrogen atom and two substituents containing substituted or unsubstituted alkyl or aryl or aliphatic or aromatic groups as defined below. The amine group may be tertiary amine, which means that nitrogen is bonded to three substituents containing substituted or unsubstituted alkyl or aryl or aliphatic or aromatic groups. The amine group can also be quaternary amine, which means that the specified amine group is bound to a fourth group, which produces a positively charged ammonium group.

It should be understood that any or all of the amines in the present invention may be in a free amine form (i.e. $-NH_2$ for primary amine) or a protonated form formed with pharmaceutically acceptable anions (i.e., $-NH_3^+Y^-$ for primary amine, where $Y^-$ is a pharmaceutically acceptable anion).

As used herein, the term "amide group" refers to a functional group containing a carbonyl attached to nitrogen. "Carbonyl" refers to a functional group containing a carbon atom bonded to an oxygen atom via a double bond, which is expressed as (C=O).

The term "alkane" refers to saturated hydrocarbon bonded via a single bond. Alkane can be linear or branched. "Cycloalkane" is a saturated hydrocarbon ring bonded via a single bond.

As used herein, the term "$C_1$-$C_6$ alkyl" refers to saturated linear or branched or cyclic hydrocarbon consisting essentially of 1 to 6 carbon atoms and a corresponding number of hydrogen atoms. Typically, straight or branched chain groups have 1 to 10 carbons, or more typically 1 to 5 carbons. Exemplary $C_1$-$C_6$ alkyl includes methyl, ethyl, n-propyl, isopropyl, n-butyl, isobutyl, and the like. According to the teachings of the present disclosure, other $C_1$-$C_6$ alkyl will be apparent to those skilled in the art.

As used herein, the term "$C_2$-$C_9$ heteroalkyl" refers to saturated linear or branched or cyclic hydrocarbon consisting essentially of 2 to 10 atoms, of which 2 to 9 atoms are carbon and the remaining atoms are selected from nitrogen, sulfur and oxygen. According to the teachings of the present disclosure, exemplary $C_2$-$C_9$ heteroalkyl will be apparent to those skilled in the art.

As used herein, the term "$C_3$-$C_{10}$ cycloalkyl" refers to a non-aromatic saturated hydrocarbon group that forms at least one ring consisting essentially of 3 to 10 carbon atoms and a corresponding number of hydrogen atoms. The $C_3$-$C_{10}$ cycloalkyl may be monocyclic or polycyclic. In addition to covalent bonds, each ring of polycyclic cycloalkyl may have different linkages, such as fused, bridged, spiro ring, and the like.

As used herein, the term "$C_2$-$C_9$ heterocycloalkyl" refers to a non-aromatic group having 3 to 10 atoms to form at least one ring, of which 2 to 9 ring atoms are carbon and the remaining atoms are selected from nitrogen, sulfur and oxygen. The $C_2$-$C_9$ heterocycloalkyl may be monocyclic or polycyclic. In addition to substitution via covalent bond, the individual rings of such polycyclic heterocycloalkyl may have different linkages, such as fused, bridged, spiro ring, and the like. According to the teachings of the present disclosure, exemplary $C_2$-$C_9$ heterocycloalkyl will be apparent to those skilled in the art.

The term "aliphatic group" or "aliphatic" refers to a non-aromatic group composed of carbon and hydrogen and may optionally include one or more double bonds and/or triple bonds. In other words, an aliphatic group is any group that is composed of carbon and hydrogen and does not contain aromatic functionality. Aliphatic groups can be linear, branched or cyclic and typically contain 1 to 24 carbon atoms.

The term "aryl group" can be used interchangeably with "aryl", "aryl ring", "aromatic", "aromatic group" and "aromatic ring". Aryl includes a carbocyclic aromatic group, which typically has 6 to 14 ring carbon atoms. Aryl also

includes heteroaryl, which typically has 5 to 14 ring atoms, of which one or more heteroatoms are selected from nitrogen, oxygen, and sulfur.

As used herein, the term "$C_6$-$C_{14}$ aryl" refers to an aromatic functional group having 6 to 14 carbon atoms to form at least one ring.

As used herein, the term "$C_2$-$C_9$ heteroaryl" refers to aromatic functional group having 5 to 10 atoms to form at least one ring, of which 2 to 9 ring atoms are carbon and the remaining ring atoms are selected from nitrogen, sulfur and oxygen. The $C_2$-$C_9$ heteroaryl can be monocyclic or polycyclic. In addition to substitution via a covalent bond, individual ring of such polycyclic heteroaryl may have different linkages, such as fused, bridged, spiro ring, and the like. The $C_2$-$C_9$ heteroaryl is typically connected to the main structure via a carbon atom. However, those skilled in the art will understand when certain other atoms, such as ring heteroatoms, are connected to the main structure. According to the teachings of the present disclosure, other $C_2$-$C_9$ heteroaryl will be apparent to those skilled in the art.

As used herein, the term "alkylamine" refers to $C_1$-$C_6$ alkyl containing a primary, secondary or tertiary amine group instead of a hydrogen atom, which is expressed as $C_1$-$C_6$ alkylamine and ($C_1$-$C_6$ alkyl)$_2$ amine.

The term "alkyl ester" refers to $C_1$-$C_6$ alkyl containing an ester group instead of a hydrogen atom, which is expressed as -O(O)C($C_1$-$C_6$ alkyl).

The term "alkyl acid" refers to $C_1$-$C_6$ alkyl containing a carboxylic acid group instead of a hydrogen atom, which is expressed as $C_1$-$C_6$ alkyl-COOH.

The term "fatty acid" refers to an acid of non-aromatic hydrocarbon, which is expressed as $C_1$-$C_6$ alkyl-COOH and $C_3$-$C_6$ alkyl-COOH.

The term "halogen" refers to fluorine (F), chlorine (Cl), bromine (Br), iodine (I) or astatine (At) ion.

The term "methoxy" refers to ($C_1$) alkyl containing oxygen instead of one hydrogen atom, which is expressed as -(O)$CH_3$.

The term "polyol" refers to alcohol containing multiple hydroxyl groups;

"Substituted" means that the carbon of alkyl, heterocyclyl, or aryl is substituted with one or more non-carbon substituents. The non-carbon substituents are selected from nitrogen, oxygen and sulfur.

"Unsubstituted" means that the group contains only hydrogen and carbon.

"Ester group" means a -C(O)O-R' group, where R' is as defined above, but R' cannot be hydrogen.

"Alkylsulfonyl" represents a group represented by the formula -$SO_2$R, and R represents alkyl.

"Sulfonyl" means -S($O_2$)-.

"Sulfonamido" means -S($O_2$)NH-.

"1,3,4-thiadiazole" means

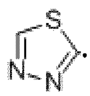.

"1,3,4-oxadiazole" means

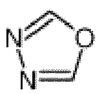.

"3-pyrroline" means

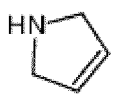.

"2-pyrazoline" means

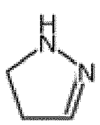.

"1,2,3-azole" means

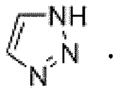

"1,2,3-triazole" means

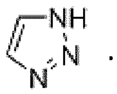

"Thiomorpholine 1,1-dioxide" means

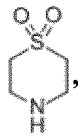

when it is used as a substituent group, it is connected to the parent structure of the compound through N, i.e.,

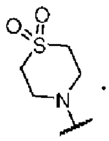

## DETAILED DESCRIPTION

The following further describes the present invention with reference to the embodiments. However, the embodiments are not intended to limit the scope of the present invention. Simple improvements to the preparation method of the present invention without departing from the idea of the present invention shall all fall within the protection scope of the present invention. The experimental methods without specific conditions in the following embodiments are usually in accordance with the well-known means. Unless otherwise specified, the test materials used in the following embodiments are purchased from conventional biochemical reagent stores.

Embodiment 1: Preparation of compound EXP-1

A synthetic route is as follows:

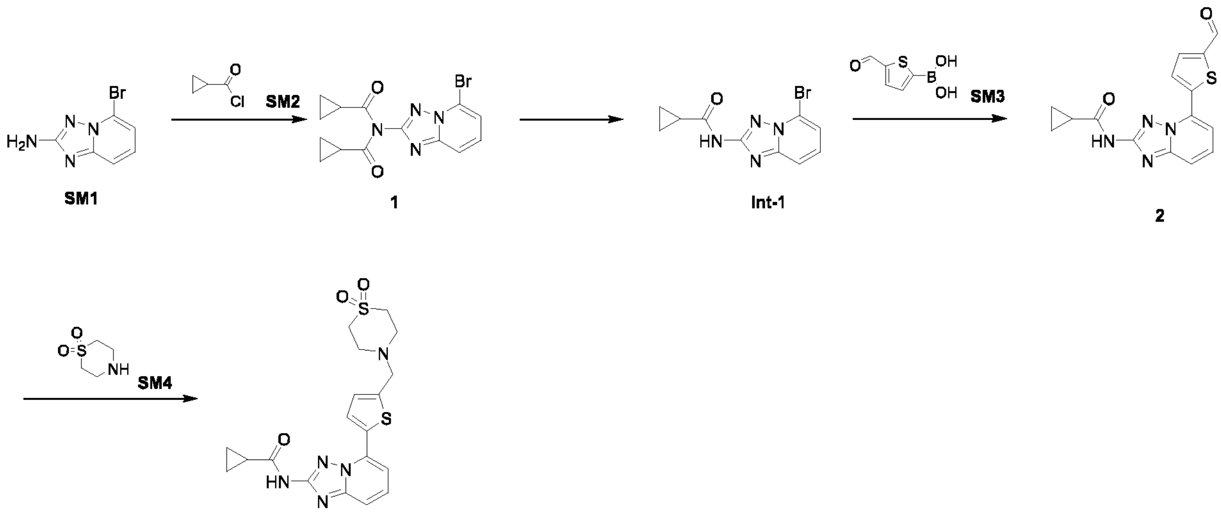


SM1 1 Int-1 2 EXP-1

Step 1.1 Preparation of compound 1

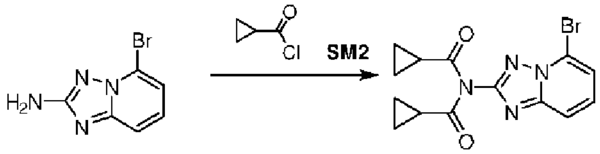


SM1 1

5-bromo-[1,2,4]triazolo[1,5-a]pyridin-2-amine (SM1) (2 g, 9.4 mmol) and triethylamine (2.4 g, 23.5 mmol) were dissolved in acetonitrile (10 mL) and cyclopropylcarbonyl chloride (SM2) (2.45 g, 23.5 mmol) was added dropwise at 0°C. The reaction was naturally warmed to room temperature and stirred for 16 h. The reaction solution was concentrated under vacuum to obtain a bisacylated intermediate N-(5-bromo-[1 ,2,4]triazolo[1 ,5-a]pyridin-2-yl)-N-(cyclopropanecarbonyl)cyclopropanecarbo xamide (1) (3.3 g crude product, yellow solid), which could be used directly in the next step without further purification.

**LCMS:**$t_R$ = 0.785 min in 5-95AB_1.5min_220&254_Shimadzu.lcm chromatography (Agilent Pursuit 5 C18 20*2.0 mm), MS (ESI) m/z = 350.9 $[M+2+H]^+$.

Step 1.2 Preparation of compound Int-1

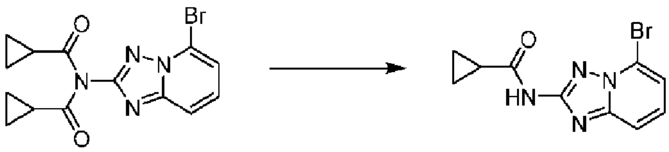

1 Int-1

N-(5-bromo-[1,2,4]triazolo[1,5-a]pyridin-2-yl)-N-(cyclopropanecarbonyl)cyclopropanec arboxamide (1) (3.3 g crude product, 9.4 mmol) was dissolved in a solution of methanol (30 mL), potassium carbonate (3.9 g, 28.2 mmol) was added and stirred for 1 h at room temperature. The reaction solution was concentrated under vacuum at room temperature, and the resulting crude product was dissolved and dispersed with ethyl acetate (250 mL) and then filtered. The filter cake was washed with dichloromethane (250 mL) and tetrahydrofuran (250 mL) sequentially. The filtrate was collected, concentrated under vacuum, and further slurried with petroleum ether/ethyl acetate (22 mL, 10:1 v/v) to obtain the product N-(5-bromo-[1,2,4]triazolo[1,5-a]pyridin-2-yl)cyclopropanecarboxamide (Int-1) (2.14 g, 80% yield, 99% purity) as a yellow solid.

**LCMS:**$t_R$ = 0.699 min in 5-95AB_1.5min_220&254_Shimadzu.lcm chromatography (Merk RP18e 25-3 mm), MS (ESI) m/z = 282.9 $[M+H+2]^+$.

**$^1$H NMR** (400MHz,DMSO-$d_6$):$\delta$=11.21 (br s, 1H), 7.71 (dd, *J*=8.4, 0.8 Hz, 1H),7.56 (t,*J*= 7.2 Hz, 1H), 7.50 (dd, *J*= 7.6, 0.6 Hz, 1H), 2.04 (br s, 1H), 0.85-0.82 (m, 4H).

Step 1.3 Preparation of compound 2

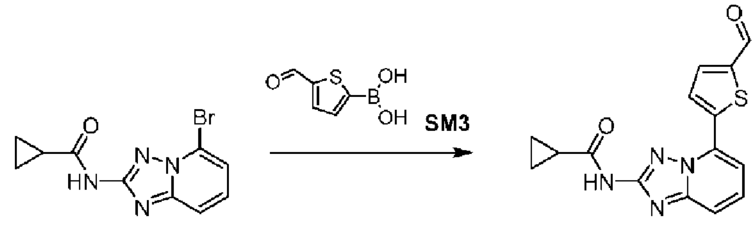


Int-1

2

N-(5-bromo-[1,2,4]triazolo[1,5-a]pyridin-2-yl)cyclopropanecarboxamide (Int-1) (300 mg, 1.1 mmol), (5-formyl-2-thienyl)boronic acid (SM3) (332.9 mg, 2.1 mmol) and potassium carbonate (442.5 mg, 3.2 mmol) were dissolved in dioxane (5 mL) and water (1 mL). 1,1-bis(diphenylphosphine)ferrocene palladium chloride (39.0 mg, 53.4 umol) was quickly added after replacing with nitrogen for three times. The reaction was replaced with nitrogen for another three times and stirred at 90°C for 30 min under nitrogen protection. The reaction solution was diluted and extracted with water (20 mL) and dichloromethane (20 mL × 3). The organic phase was washed with saturated saline solution (20 mL), dried over anhydrous sodium sulfate and filtered, and the filtrate was concentrated under vacuum to obtain a crude product, which was purified by silica gel column chromatography (eluent: 1%-2% methanol/dichloromethane) to obtain the product N-[5-(5-formyl-2-thienyl)-[1,2,4]triazolo[1, 5-a]pyridin-2-yl]cyclopropanecarboxamide (2) (350 mg semi-pure product containing part of raw material 1, calculated yield 49.4%) as a yellow solid.

**LCMS:**$t_R$= 0.783 min in 5-95AB_1.5min_220&254_Shimadzu.lcm chromatography (Agilent Pursuit 5 C18 20*2.0 mm), MS (ESI) m/z = 312.8 $[M+H]^+$.

Step 1.4 Preparation of compound EXP-1

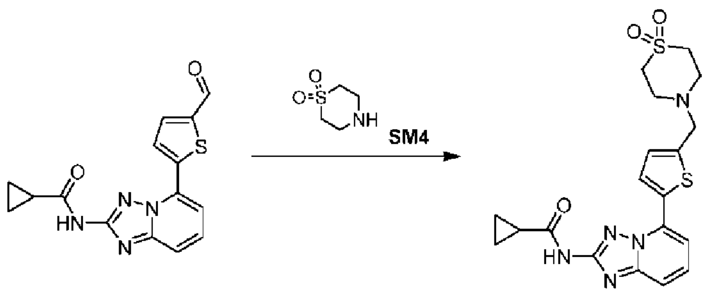


2

EXP-1

N-[5-(5-formyl-2-thienyl)-[1,2,4]triazolo[1,5-a]pyridin-2-yl]cyclopropanecarboxamide (2) (220 mg semi-pure, 331.0 umol) and 1,1-dioxothiomorpholine (SM4) (53.7 mg, 397.3 umol) were dissolved in methanol (6 mL) and the pH was adjusted to about 6 by adding acetic acid dropwise. The reaction was stirred at 50°C for 1 h. After cooling to room temperature, sodium cyanoborohydride (41.6 mg, 662.1 umol) was added and stirring was continued at 30°C for 15 h. The reaction solution was treated with saturated sodium bicarbonate aqueous solution (3 mL), diluted with water (10 mL) and extracted with dichloromethane (20 mL × 3). The organic phases were combined and then washed with saturated saline solution (20 mL), dried over anhydrous sodium sulfate and filtered, and the filtrate was concentrated under vacuum to obtain a crude product, which was isolated and purified under alkaline preparative condition to obtain the target compound EXP-1 (28.9 mg, 20% yield) as a white solid.

**LCMS:**$t_R$ = 1.893 min in 10-80AB_7min_220&254_Shimadzu.lcm chromatography (Xtimate C18 2.1*30 mm), MS (ESI) m/z = 432.0 $[M+H]^+$.

**HPLC:**$t_R$ = 2.50 min in 10-80AB_8min_Shimadzu.lcm chromatography (Ultimate 3.0*50 mm 3 um).

**$^1$H NMR** (400 MHz, $CDCl_3$):δ=8.77 (s, 1H), 8.12 (d, *J*=3.6 Hz, 1H), 7.61-7.51 (m, 2H), 7.37 (dd, J= 6.4, 2.8 Hz, 1H), 7.07 (d, *J*=3.6 Hz, 1H), 3.96 (s, 2H), 3.18-3.06 (m, 8H), 1.66-1.58 (m, 1H), 1.30-1.20 (m, 2H), 1.03-0.93 (m, 2H).

The following embodiment compounds of Table 1 were prepared according to the same method as in the above embodiment, using commercially available compounds or by reference to the preparation method of the intermediate compounds shown.

[Table 1]

| No. | Struture | H-NMR or LC/MS |
|---|---|---|
| EXP-2 | 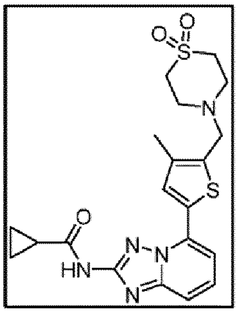 | **LCMS:**$t_R$ = 2.586 min in 10-80AB_4min_220&254_ Shimadzu.lcm, chromatography (Xtimate C18 2.1 *30 mm), MS (ESI) m/z =446.3 [M+H]$^+$. **HPLC:** $t_R$ = 2.51 min in 10-80CD_8min_met, chromatog raphy (XBridge Shield RP18, 5 um). **1H NMR** (400 MHz, DMSO-$d_6$): δ= 11.15 (brs, 1H), 8.13 (s, 1H), 7.72-7.58 (m, 3H), 3.91 (s, 2H), 3.15-3.13 (m, 4H), 3.01-2.99 (m, 4H), 2.26 (s, 3H), 2.20- .13 (m,1H), 0.89-0.86 (m, 4H). |

Embodiment 2: Preparation of compound EXP-3

A synthetic route is as follows:

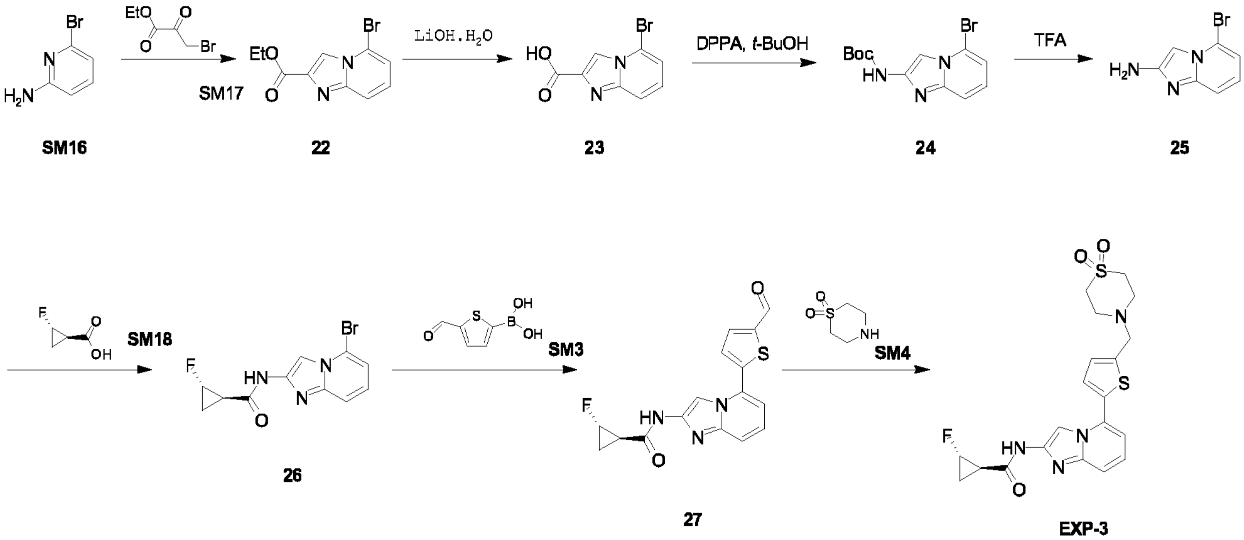


SM16 22 23 24 25

26 27 EXP-3

Step 2.1 Preparation of compound 22

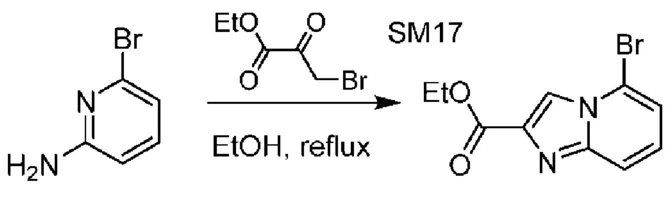


SM16 22

6-bromopyridin-2-amine (SM16) (5 g, 28.90 mmol) and ethyl bromopyruvate (SM17) (6.20 g, 31.79 mmol) were dissolved in ethanol (30 mL) and stirred at 85°C for 16 h. The reaction solution was filtered, and the filter cake was collected and slurried with petroleum ether/ethyl acetate (5/1, v/v, 50 mL). The solid was filtered and dried to obtain ethyl 5-bromoimidazo[1,2-a]pyridine-2-carboxylate (22) (8.41 g, 83% yield, hydrobromide, yellow solid).

**1H NMR** (400 MHz, DMSO-$d_6$): δ = 8.56 (s, 1H), 7.75 (d, *J* = 8.8 Hz, 1H), 7.52-7.44 (m,2H), 4.36 (q, *J*= 7.2 Hz,

2H), 1.34 (t, *J*= 7.2 Hz, 3H).

Step 2.2 Preparation of compound 23

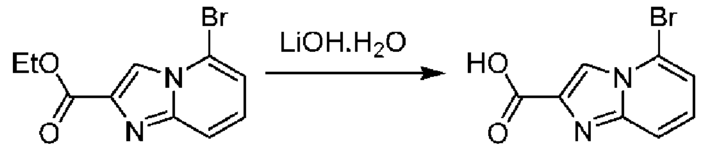


**22** **23**

Ethyl 5-bromoimidazo[1,2-a]pyridine-2-carboxylate (22) (2 g, 5.71 mmol, hydrobromide) was dissolved in a solvent containing 5 ml water, 5 ml tetrahydrofuran and 5 ml methanol, and lithium hydroxide monohydrate (839 mg, 20.00 mmol) was added. The reaction solution was stirred at 15°C for 2 h. The reaction solution was added with water (10 mL) and dichloromethane (20 mL). The aqueous phase was adjusted to pH=5 with dilute hydrochloric acid (1 N), filtered and the filter cake was dried to obtain 5-bromoimidazo[1,2-a]pyridine-2-carboxylic acid (23) (1.24 g, 90% yield, white solid) for the next step.

**LCMS:** $t_R$= 0.292 min in 5-95AB_1.5min_220&254_Shimadzu.lcm, chromatography (Chromolith Flash RP-18, 5 um, 3.0*25 mm), MS (ESI) m/z = 241.1[M+H]$^+$.

**1H NMR** (400 MHz, DMSO-*d*$_6$):δ= 8.37 (s, 1H), 7.72 (d, *J*=8.8 Hz, 1H), 7.43 (dd, *J*= 8.8, 1.2 Hz, 1H), 7.39-7.28 (m, 1H).

Step 2.3 Preparation of compound 24

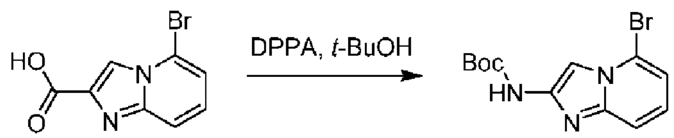


**23** **24**

5-bromoimidazo[1,2-a]pyridine-2-carboxylic acid (23) (1.24 g, 5.14 mmol), 4 A molecular sieve (2.48 g, 10.29 mmol) and triethylamine (1.56 g, 15.43 mmol) were suspended in tert-butanol (12 mL) and diphenyl azidophosphate (2.12 g, 7.72 mmol) was added. The reaction solution was stirred under nitrogen atmosphere at 90°C for 16 h. The reaction solution was filtered, the filtrate was concentrated under vacuum and a crude product was purified by silica gel chromatography (methanol/dichloromethane, from 0% to 2%) to obtain tert-butyl 5-bromoimidazo[1,2-a]pyridine-2-aminocarboxylate (24) (1.01 g, 49% yield, 78% purity, yellow solid).

**LCMS:**$t_R$ = 1.443 min in 10-80AB_2min_220&254_Shimadzu.lcm, chromatography (Xtimate C18 2.1 *3 mm, 3 um), MS (ESI) m/z = 312.1 [M+H]$^+$.

Step 2.4 Preparation of compound 25

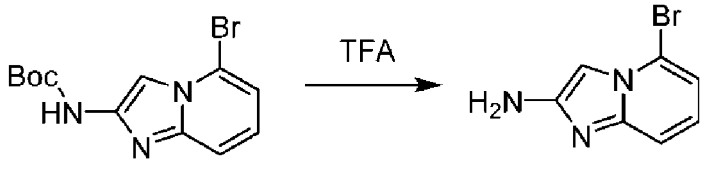


**24** **25**

Tert-butyl 5-bromoimidazo[1,2-a]pyridine-2-aminocarboxylate (24) (1.01 g, 3.24 mmol) was dissolved in dichloromethane (20 mL), and trifluoroacetic acid (12.32 g, 108.05 mmol) was added. The reaction solution was stirred at 15°C for 2 h. The reaction solution was concentrated, adjusted to pH = 9 with saturated sodium bicarbonate, and then extracted with dichloromethane (30 mL × 3). The organic phases were combined, washed with saline solution (20 mL), dried over anhydrous sodium sulfate, filtered and concentrated to obtain a crude product, which was purified by silica gel chromatographic column (methanol/dichloromethane, from 0% to 5%) to obtain 5-bromoimidazo[1,2-a]pyridin-2-amine (25) (400 mg, 58% yield, yellow solid).

$^1$**H NMR** (400 MHz,DMSO-$d_6$):δ= 7.24 (d, *J*=8.0 Hz,1H), 7.06 (d, *J*=7.2 Hz, 1H), 7.02-6.97 (m, 2H), 5.30 (brs, 2H).

Step 2.5 Preparation of compound 26

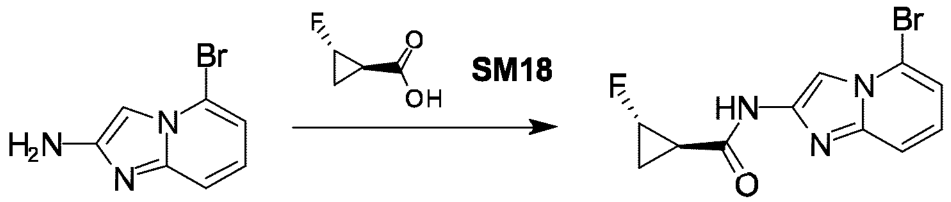


**25** **26**

Trans-2-fluorocyclopropylcarboxylic acid (SM18) (491 mg, 4.72 mmol) was dissolved in dichloromethane (1 ml) and oxalyl chloride (539 mg, 4.24 mmol) and a drop of dimethylformamide were added at 0°C. The reaction solution was stirred at 0°C for 1 h, and then a solution of 5-bromoimidazo[1,2-a]pyridin-2-amine (25) (500 mg, 2.36 mmol) in dimethylacetamide (4 mL) was added at 0°C and stirred at room temperature for 2 h. The reaction solution (combined with batch EB4-106, with the addition of 100 mg of substrate 1) was quenched by adding saturated sodium bicarbonate solution (40 mL) and extracted with ethyl acetate (60 mL × 3). The organic phases were combined, washed with saline solution (40 mL), dried over anhydrous sodium sulfate, filtered, concentrated under vacuum and purified by silica gel chromatographic column (methanol/dichloromethane, from 0% to 2%) to obtain trans-*N*-(5-bromoimidazo[1,2-a]pyridin-2-yl)-2-fluoro-cyclopropylcarboxamide (26) (587 mg, 69% yield, yellow solid).

$^1$**H NMR** (400 MHz,DMSO-*$d_6$*): δ= 11.31 (s, 1H), 8.02 (s, 1H), 7.53 (d, *J*=8.8 Hz, 1H), 7.32-7.17 (m, 2H), 5.07-4.75 (m, 1H), 2.47-2.41 (m, 1H), 1.61-1.47 (m, 1H), 1.30-1.20 (m, 1H).

Step 2.6 Preparation of compound 27

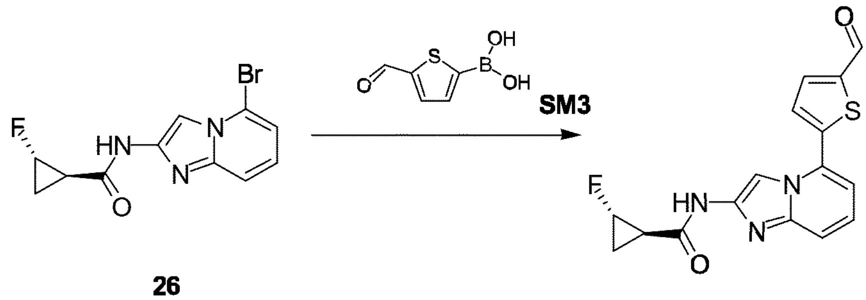


26

27

(5-formyl-2-thiophene)boronic acid (SM3) (419 mg, 2.68 mmol), trans-*N*-(5-bromoimidazo[1,2-a]pyridin-2-yl)-2-fluoro-cyclopropylcarboxamide (26) (200 mg, 0.671 mmol) and potassium carbonate (278 mg, 2.01 mmol) were dissolved in dioxane (4 mL) and water (0.5 mL), and the reaction was replaced with nitrogen for three times. Then 1,1-bis(diphenylphosphine)ferrocene palladium chloride (49 mg, 0.0671 mmol) was added, and the reaction was replaced with nitrogen for another three times. The reaction solution was stirred at 100°C for 1 h. The reaction solution was concentrated and purified by silica gel chromatographic column (methanol/dichloromethane, from 0% to 2%) to obtain trans-2-fluoro-*N*-[5-(5-formyl-2-thiophene)imidazo[1,2-a]pyridin-2-yl]cyclopropylcarboxami de (27) (90 mg, 41% yield, 100% purity, yellow solid).

**LCMS:**$t_R$= 0.832 min in 5-95AB_1.5min_220&254_Shimadzu.lcm, chromatography (Xtimate C18 2.1 *30 mm, 3 um), MS (ESI) m/z = 330.1 $[M+H]^+$.

Step 2.7 Preparation of compound EXP-3

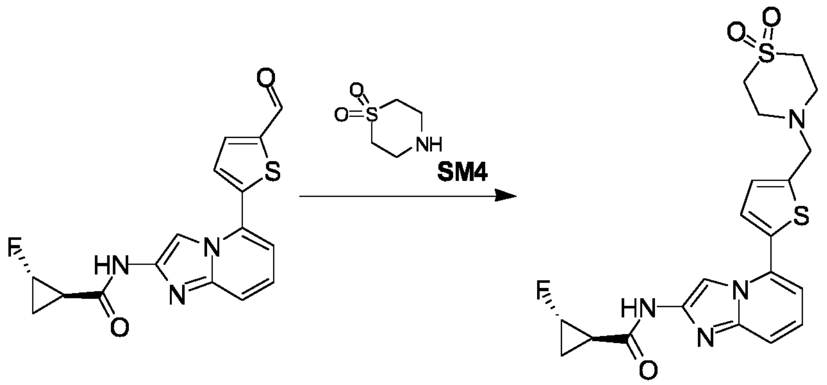


27

EXP-3

Trans-2-fluoro-*N*-[5-(5-formyl-2-thiophene)imidazo[1,2-a]pyridin-2-yl]cyclopropylcarbo xamide (27) (90 mg, 0.273 mmol) and thiomorpholine-1,1-dioxide (SM4) (55 mg, 0.410 mmol) were dissolved in methanol (4 mL), dichloromethane (2 mL) and tetrahydrofuran (2 ml). Acetic acid (21 mg, 0.355 mmol) was added and stirred at room temperature for 2 h. Sodium cyanoborohydride (69 mg, 1.09 mmol) was added and the reaction solution was stirred at room temperature for 16 h. The reaction solution was quenched by adding saturated sodium bicarbonate (30 mL), stirred for half an hour at room temperature, and extracted by adding dichloromethane (50 mL × 3). The organic phases were combined, washed with saline solution (30 mL), dried over anhydrous sodium sulfate, filtered, and concentrated under vacuum, and a crude product was purified by preparative HPLC (Column: Waters Xbridge 150*25 mm*5 um; Condition: water (0.04% $NH_3.H_2O$+10 mM $NH_4HCO_3$)-ACN; Begin B 25%; End B, 55%; Gradient Time: (8 min); Flow Rate: (25 mL/min))to obtain trans-*N*-[5-[5-[(1,1-dioxo-1,4-thiazin-4-yl)methyl]-2-thiophene]imidazo[1,2-a]pyridin-2-yl]-2 -fluoro-cyclopropylcarboxamide (EXP- 3) (22.4 mg, 17% yield, 96.17% purity, yellow solid).

**LCMS:t$_R$**= 1.765 min in 10-80AB_4min_220&254_Shimadzu.lcm, chromatography (Xtimate C18 2.1 *3 mm, 3 um), MS (ESI) m/z = 449.2 $[M+H]^+$.

**HPLC:**$t_r$= 2.36 min in10-80AB_8min_215&220&254.met.

**$^1$H NMR** (400 MHz,DMSO-$d_6$): δ= 11.25 (s, 1H), 8.30 (s, 1H), 7.56 (d, *J*=3.6 Hz, 1H), 7.49(d, *J*= 9.2 Hz, 1H),

7.32 (dd, *J*= 9.2, 7.2 Hz, 1H), 7.20 (d, *J*=3.6 Hz, 1H), 7.07 (d, *J*=7.2 Hz, 1H), 5.3- 4.70 (m, 1H), 4.01 (s, 2H), 3.17-3.10 (m, 4H), 3.03-2.93 (m, 4H), 2.48-2.40 (m, 1H), 1.62-1.45 (m, 1H), 1.28-1.16 (m, 1H).

Embodiment 3: Preparation of compound EXP-4

A synthetic route is as follows:

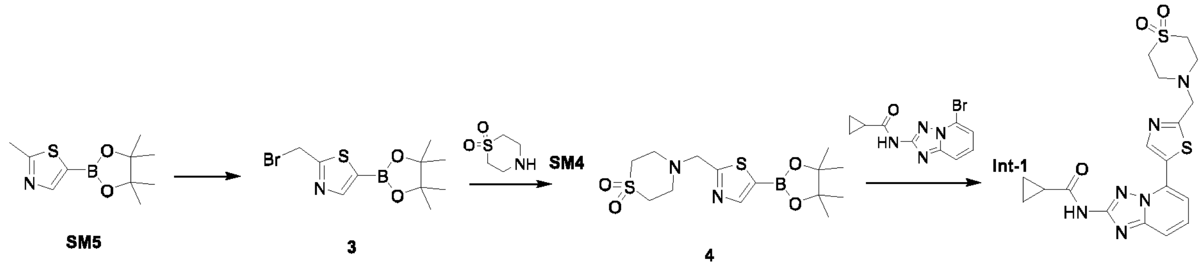


SM5 3 4 EXP-4

Step 3.1 Preparation of compound 3

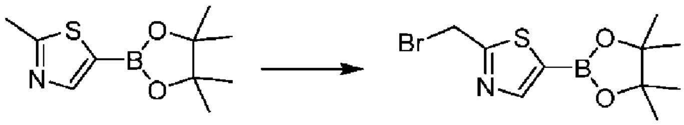

SM5 3

2-methylthiazole-5-boronic acid pinacol ester (SM5) (100 mg, 0.444 mmol) was dissolved in carbon tetrachloride (2 mL) and *N*-bromosuccinimide (103 mg, 0.577 mmol) and azodiisobutyronitrile (7.3 mg, 0.0444 mmol) were added. The reaction solution was stirred at 80°C for 0.5 h under nitrogen protection. The reaction solution was filtered, and the filtrate was concentrated under vacuum to obtain a crude product, which was then dissolved in tetrahydrofuran (2 mL). Diisopropylethylamine (0.1 mL) and diethyl phosphite (74 uL) were added sequentially and stirred at 10°C for 25 min to obtain 2-bromomethylthiazole-5-boronic acid pinacol ester (3) (135 mg, crude product, brown solution dissolved in 2 mL tetrahydrofuran), which was used directly in the next step.

**LCMS:$t_R$**= 0.590 min in 5-95AB_1.5min_220&254_Shimadzu.lcm chromatography (Merk RP18e 25-3 mm), MS (ESI) m/z= 221.7 $[M-81]^+$.

Step 3.2 Preparation of compound 4

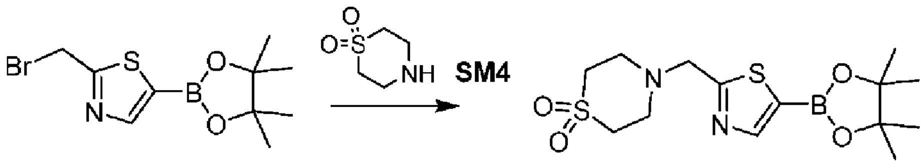


3 4

2-bromomethylthiazole-5-boronic acid pinacol ester (3) (135 mg, 0.444 mmol) and potassium carbonate (246 mg, 1.78 mmol) were suspended in tetrahydrofuran (2 mL) and thiomorpholine-1,1-dioxide (SM4) (72 mg, 0.533 mmol) was added. The reaction solution was reacted at 10°C for 24 h under nitrogen protection. The reaction solution was concentrated under vacuum to obtain 4-[[5-(4,4,5,5-tetramethyl-1,3,2-dioxaborolane-2-)thiazol-2-]methyl]-1,4-thiazine 1,1-dioxide (4) (159 mg, crude product, red solid), which was used directly in the next step.

**LCMS:$t_R$**= 0.287 min in 5-95AB_1.5min_220&254_Shimadzu.lcm chromatography (Merk RP18e 25-3 mm), MS (ESI) m/z= 276.8 $[M-81]^+$.

Step 3.3 Preparation of compound EXP-4

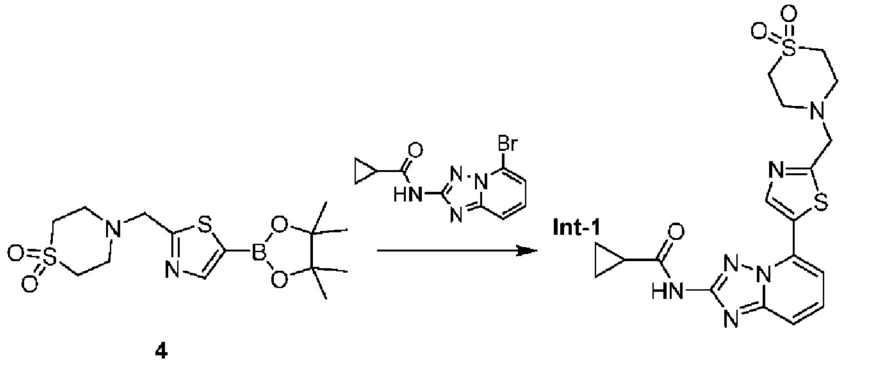


4

EXP-4

N-(5-bromo-[1,2,4]triazolo[1,5-a]pyridin-2-yl)cyclopropanecarboxamide (Int-1) (20 mg, 0.071 mmol), 4-[[5-(4,4,5,5-tetramethyl-1,3,2-dioxaborolan-2-)thiazol-2-]methyl]-1,4-thiazine 1,1-dioxide **(4)** (76.5 mg, 0.213 mmol), and potassium carbonate (29.5 mg, 0.213 mmol) were suspended in dioxane (1 mL) and water (0.2 mL), and the reaction solution was replaced with nitrogen for three times. Then 1,1-bis(tert-butylphosphine)ferrocene palladium chloride (9.3 mg, 0.014 mmol) was added quickly, and the reaction solution was replaced with nitrogen for another three times and stirred at 85°C for half an hour. The reaction solution was concentrated under vacuum and quickly purified by a column (eluent: 0%-0.5% methanol/dichloromethane) to obtain a crude product, which was isolated under alkaline condition to obtain the target molecule *N*-[5-[2-[(1,1-dioxo-1,4-thiazine-4-)methyl]thiazol-5-]-[1,2,4]triazolo[1,5-a]pyridine-2-]cyclo propylcarboxamide (EXP-4) (5.6 mg, 6% yield, 93% purity, yellow solid).

**LCMS:** $t_R$= 1.793 min in 10-80AB_7min_220&254_Shimadzu.lcm, chromatography (Xtimate C18, 2.1*30 mm), MS (ESI) m/z = 433.0 $[M+H]^+$.

**HPLC:** $t_R$ = 2.54 min in 10-80AB_8min.met (Ultimate C18 3*50 mm 3 um).

**$^1$H NMR** (400 MHz,DMSO-$d_6$): δ= 11.22 (br s, 1H), 8.93 (s, 1H), 7.81-7.63 (m, 3H), 4.16 (s, 2H), 3.23-3.04 (m, 8H), 2.13-2.06 (m, 1H), 0.95-0.81 (m, 4H).

Embodiment 4: Preparation of compound EXP-5

A synthetic route is as follows:

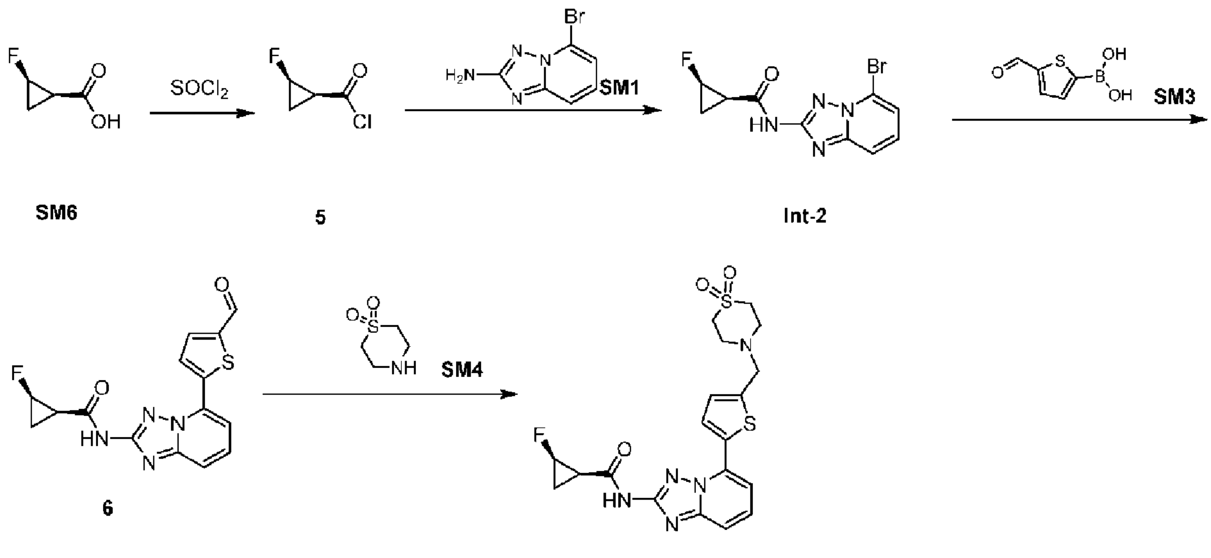


SM6 5 Int-2

6

EXP-5

Step 4.1 Preparation of compound 5

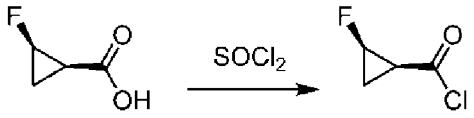


SM6 5

Under nitrogen protection, cis-2-fluorocyclopropanecarboxylic acid (SM6) (50 mg, 0.48 mmol) was dissolved in $SOCl_2$ (1 mL) and stirred for 1 h at 75°C. The reaction was concentrated under reduced pressure below 30°C to obtain the compound cis-2-fluorocyclopropanecarbonyl chloride (5) (58 mg, 98% yield) as yellow oil, which was used directly in the next step.

Step 4.2 Preparation of compound Int-2

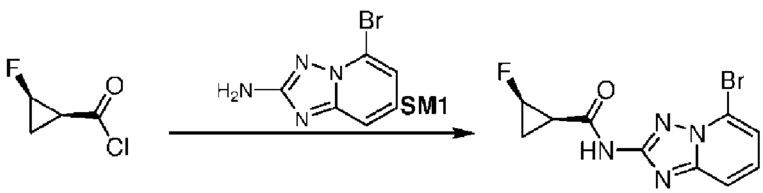


5 Int-2

Under nitrogen protection, 5-bromo-[1,2,4]triazolo[1,5-a]pyridin-2-amine (SM1) (100 mg, 0.469 mmol) and cis-2-fluorocyclopropanecarbonyl chloride (5) (58 mg, 0.469 mmol) were dissolved in pyridine (1 mL), heated to 60°C and stirred for 1 h. The reaction was concentrated to obtain a crude product, which was dissolved in dichloromethane (3 mL) and methanol (1 mL). The insoluble solid was removed via filtration, the residue was washed with dichloromethane (1 mL x 2), and the filtrate was concentrated to obtain a crude product N-[5-bromo-[1,2,4]triazolo[1,5-a]pyridin-2-yl]-cis-2-fluorocyclopropanecarboxamide (Int-2) (130 mg, crude product) as a yellow solid, which was used directly in the next step.

**LCMS:$t_R$**= 0.609 min in 5-95AB_1.5min_220&254_Shimadzu.lcm, chromatography (Agilent Pursuit 5 C18 20*2.0 mm), MS (ESI) m/z =300.6[M+H]$^+$.

**$^1$H NMR** (400 MHz, $CDCl_3$): δ= 8.01 (s, 1H), 7.65 (d, *J*=8.8 Hz, 1H), 7.40 (d, *J*=8.4 Hz, 1H), 7.25 - 7.24 (m, 1H), 4.95 - 4.76 (m, 1H), 2.08 - 1.94 (m, 1H), 1.89 - 1.77 (m, 1H), 1.26 - 1.21 (m, 1H).

Step 4.3 Preparation of compound 6

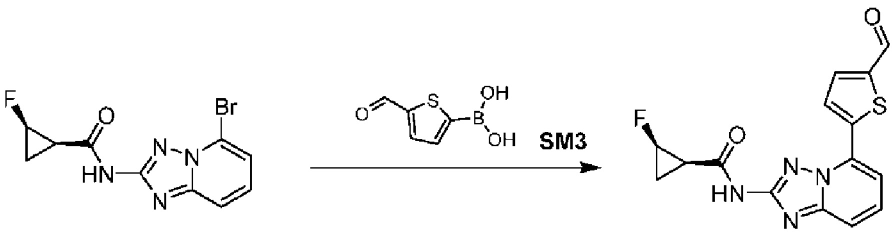


Int-2 6

*N*-[5-bromo-[1,2,4]triazolo[1,5-a]pyridin-2-yl]-cis-2-fluorocyclopropanecarboxamide (Int-2) (100 mg, 0.334 mmol), (5-formyl-2-thiophene)boronic acid (SM3) (261 mg,1.67 mmol), and sodium carbonate (106 mg, 1.00 mmol) were dissolved in tetrahydrofuran ( 2 mL) and water (1 mL). After replacing with nitrogen for three times, 1,1-bis(diphenylphosphine)ferrocene palladium chloride (47 mg, 0.067 mmol) was added. After replacing with nitrogen for three times, the reaction was raised to 100°C and stirred for 0.5 h. The reaction solution was stirred by adding water (20 mL) and extracted with ethyl acetate (40 mL). The organic phase was washed with saturated saline solution (10 mL), dried over anhydrous sodium sulfate and filtered, and the filtrate was concentrated under vacuum to obtain a crude product *N*-[5-(2-formyl)-thiophene-[1,2,4]triazolo[1,5-a]pyridin-2-yl]-cis-2-fluoro-cyclopropanecarbo xamide (6) (100 mg, crude product) as a yellow solid, which was used directly in the next step.

Step 4.4 Preparation of compound EXP-5

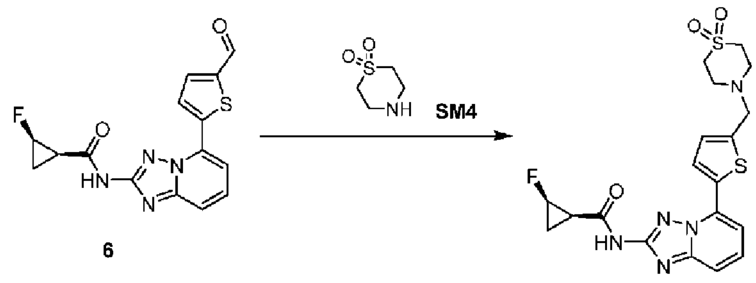


*N*-[5-(2-formyl)-thiophene-[1,2,4]triazolo[1,5-a]pyridin-2-yl]-cis-2-fluoro-cyclopropanec arboxamide (6) (100 mg, 0.302 mmol) and 1,1-dioxothiomorpholine (SM4) (82 mg, 0.605 mmol) were dissolved in methanol (5 mL). Acetic acid (9 mg, 0.151 mmol) was added, and then heated to 80°C and reacted for 0.2 h. The reaction was cooled to room temperature and sodium cyanoborohydride (152 mg, 2.42 mmol) was added, and then heated to 80°C and reacted for 0.5 h. The reaction was cooled; saturated sodium bicarbonate (10 mL) was added and stirred at room temperature for 0.5 h. Most of the methanol was removed via concentration, and then the residue was extracted with ethyl acetate (20 mL × 2), concentrated, dissolved in methanol (5 mL), isolated and purified under preparative condition to obtain the title product *N*-[5-[4-[(1,1-dioxothiomorpholine)methyl]thienyl]-[1,2,4] triazolo[1,5-a]pyridin-2-yl]-cis-2-fluoro-cyclopropanecarboxamide (EXP-5) (10.3mg, 8%yield) as a white solid.

**LCMS:**$t_R$ = 1.545 min in 10-80AB_4min_220&254_Shimadzu.lcm, chromatography (Xtimate C18 2.1*30 mm), MS (ESI) m/z=450.0[M+H]$^+$.

**HPLC:**$t_R$=2.43min in 10-80AB_8min_met, chromatography (Ultimate 3.0*50 mm*3 um).

**$^1$H NMR** (400 MHz,DMSO-$d_6$):δ=11.23 (brs, 1H), 8.26(brs, 1H), 7.77-7.56 (m, 3H), 7.20 (brs, 1H), 5.07-4.91 (m, 1H), 4.00 (brs, 2H), 3.14-2.98 (m, 8H), 2.34-2.31 (m, 1H), 1.73-1.68 (m, 1H), 1.22-1.20 (m, 1H).

The following embodiment compounds of Table 2 were prepared according to the same method as in the above embodiment, using commercially available compounds or by reference to the preparation method of the intermediate compounds shown.

[Table 2]

| No. | Structure | H-NMR or LC/MS |
|---|---|---|
| EXP-6 | 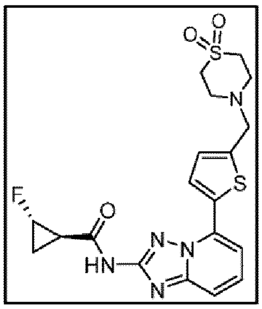 | **LCMS:$t_R$**= 1.684 min in 10-80AB_4min_220&254_Shimadzu.lcm, chromatography (Xtimate C18 2.1 *30 mm), MS (ESI) m/z =471.9[M+Na]$^+$. **HPLC:**$t_R$ = 2.67 min in 10-80AB_8min_met, chromatography(Ultimate 3.0*50 mm*3 um). **1H NMR** (400 MHz, DMSO-$d_6$): δ = 11.35 (brs, 1H), 8.26 (brs, 1H), 7.71-7.65 (m, 3H), 7.20 (s, 1H), 5.04-4.88 (m, 1H), 4.01 (s, 2H), 3.15 (s, 4H), 2.98 (s, 4H), 1.62-1.56 (m, 1H), 1.32-1.24 (m, 2H). |

(continued)

| No. | Structure | H-NMR or LC/MS |
|---|---|---|
| EXP-7 | 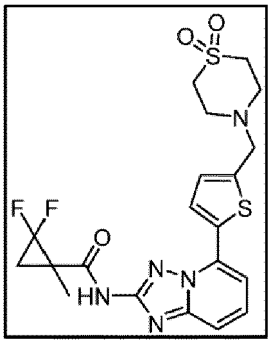 | **LCMS:** $t_R$ = 1.796 min in 10-80AB_4min_220&254_Shimadzu.lcm, chromatography (Xtimate C18 2.1 *30 mm), MS (ESI) m/z =482.1 $[M+H]^+$. **HPLC:** $t_R$ = 2.89 min in 10-80AB_8min_met, chromatography (Ultimate 3.0*50 mm*3 um). **1H NMR** (400 MHz, DMSO-$d_6$) δ = 8.28 (d, *J*=3.2 Hz, 1H), 7.75-7.65 (m, 3H), 7.22 (d, *J*=4.4 Hz, 1H), 4.02 (s, 2H), 3.16 (s, 4H), 3.00 (s, 4H), 2.15-2.13 (m, 1H), 1.58 (s, 3H), 1.58-1.57 (m, 1H). |
| EXP-8 | 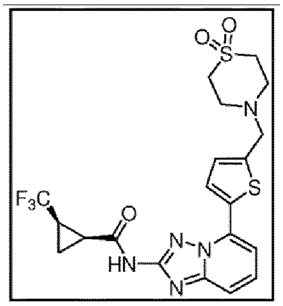 | **LCMS:** $t_R$ = 1.756 min in 10-80AB_4min_220&254_Shimadzu.lcm, chromatography (Xtimate C18 2.1 *30 mm), MS (ESI) m/z =522.1 $[M+Na]^+$. **HPLC:** $t_R$ = 2.94 min in 10-80AB_8min_met, chromatography (Ultimate 3.0*50 mm*3 um). **1H NMR** (400 MHz, DMSO-$d_6$) δ = 11.38 (brs, 1H), 8.26 (s, 1H), 7.71-7.62 (m, 3H), 7.21 (d, *J*=3.6 Hz, 1H), 4.01 (s, 2H), 3.14 (s, 4H), 2.99 (s, 4H), 2.33-2.31 (m, 2H), 1.59-1.57 (m, 1H), 1.37-1.35 (m, 1H). |
| EXP-9 | 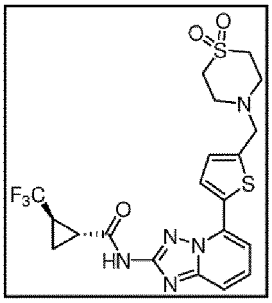 | **LCMS:** $t_R$ = 1.984 min in 10-80AB_4min_220&254_Shimadzu.lcm, chromatography (Xtimate C18 2.1 *30 mm), MS (ESI) m/z =500.0$[M+H]^+$. **HPLC:** $t_R$ = 3.26 min in 10-80AB_8min_met, chromatography (Ultimate 3.0*50 mm*3 um). **1H NMR** (400 MHz, DMSO-$d_6$)δ = 11.47 (brs, 1H), 8.25 (d, *J*=3.2 Hz, 1H), 7.74-7.63 (m, 3H), 7.19 (d, *J*= 4.0 Hz, 1H), 4.00 (s, 2H), 3.15 (s, 4H), 2.98 (s, 4H), 2.33-2.38 (m, 2H), 1.38-1.35 (m, 2H). |
| EXP-10 | 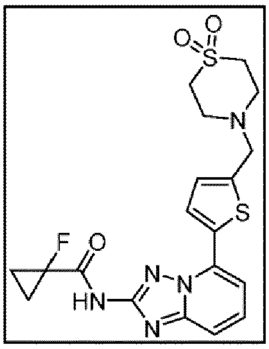 | **LCMS:** $t_R$ = 2.542 min in 10-80AB_4min_220&254_Shimadzu.lcm, chromatography (Xtimate C18 2.1 *30 mm), MS (ESI) m/z =450.2$[M+H]^+$. **HPLC:** $t_R$ = 2.39 min in 10-80AB_8min_met, chromatography (XBridge Shield RP18 5 um). **1H NMR**(400 MHz, DMSO-$d_6$): δ = 11.16 (brs, 1H), 8.27 (d, *J*= 4.0 Hz, 1H), 7.76-7.69 (m, 3H), 7.22 (d, *J*=3.6 Hz, 1H), 4.02 (s, 2H), 3.16-3.14 (m, 4H), 3.00-2.98 (m, 4H), 1.55- 1.42(m, 2H), 1.41-1.34 (m, 2H). |

(continued)

| No. | Structure | H-NMR or LC/MS |
|---|---|---|
| EXP-11 | 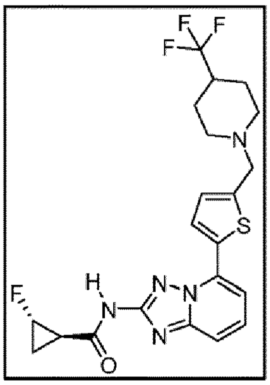 | **LCMS:** $t_R$ = 2.087 min in 10-80AB_7min_220&254_Shimadzu.lcm, chromatography (Xtimate C18 2.1*3 mm, 3 um), MS (ESI) m/z = 468.3 $[M+H]^+$. **HPLC:** $t_R$ = 2.53 min in 50-100AB_8min_220&254.met **1H NMR** (400 MHz, DMSO-$d_6$): $\delta$ = 11.31 (br s, 1H), 8.25 (d, *J*= 3.6 Hz, 1H), 7.73-7.59 (m, 3H), 7.15 (d, *J*= 3.6 Hz, 1H), 5.08-4.81 (m, 1H), 3.78 (s, 2H), 3.04-2.92 (m, 2H), 2.32-2.21 (m, 1H), 2.10-1.99 (m, 2H), 1.84-1.74 (m, 2H), 1.65-1.41 (m, 3H), 1.36-1.25 (m, 1H). |

Embodiment 5: Preparation of compound EXP-12

A synthetic route is as follows:

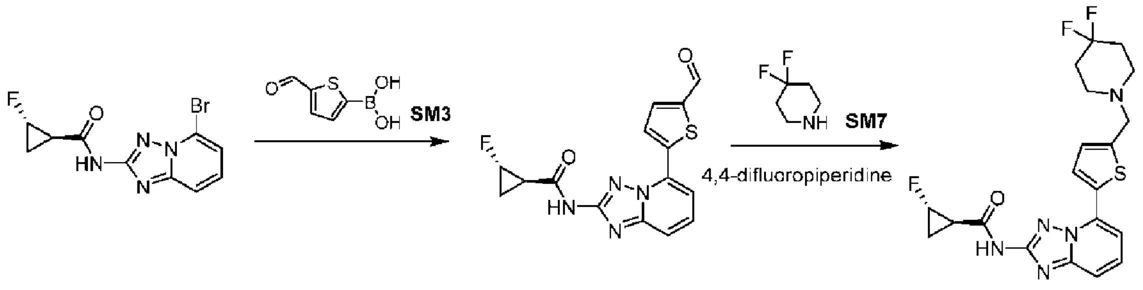


Int-3 7 EXP-12

Step 5.1 Preparation of compound 7

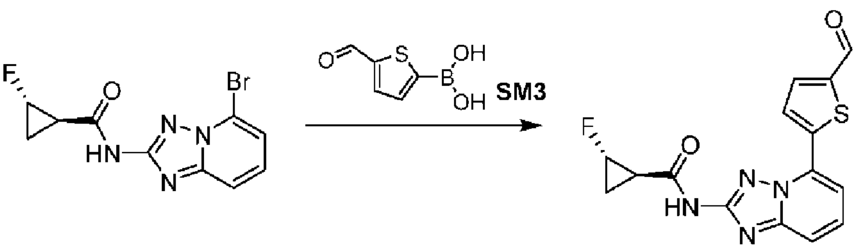


Int-3 7

Trans-*N*-(5-bromo-[1,2,4]triazolo[1,5-a]pyridin-2-yl)-2-fluoro-cyclopropanecarboxamide (Int-3) (100 mg, 0.33 mmol), (5-formyl-2-thienyl)boronic acid (SM3) (111 mg, 0.71 mmol), potassium carbonate (98 mg, 0.71 mmol), bis(triphenylphosphonium)palladium dichloride (50 mg, 0.07 mmol) were dissolved in water (0.5 mL) and tetrahydrofuran (1 mL). The reaction was replaced with nitrogen for three times and stirred at 100°C for 1 h. The reaction was filtered and the filter cake was dried to obtain a crude product, which was purified by column separation (dichloromethane:methanol= 100: 1 - 20: 1) to obtain trans-*N*-(5 -(5-formyl-2-thienyl)-[1,2,4]triazolo[1,5-a]pyridin-2-yl]-2-fluoro-cyclopropanamide (7) (50 mg, crude product, yellow solid).

**LCMS:$t_R$**= 2.054 min in 10-80AB_4.0 min_220&254_Shimadzu.lcm, chromatography (Xtimate C18, 3 um, 2.1*30 mm), MS (ESI) m/z =301.0[M+H]$^+$.

Step 5.2 Preparation of compound EXP-12

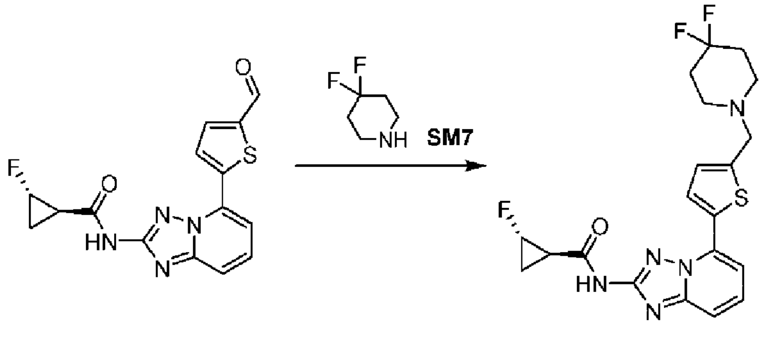


7 EXP-12

Trans-*N*-(5 -(5-formyl-2-thienyl)-[1,2, 4]triazolo[1,5-a]pyridin-2-yl]-2-fluoro-cyclopropanamide (7) (25 mg, 0.08 mmol) and 4,4-difluoropiperidine (SM7) (18 mg, 0.15 mmol) were dissolved in methanol (3 ml), then acetic acid (2 mg, 0.04 mmol) was added and stirred at 50°C for 0.5 h. Then sodium cyanoborohydride (38 mg, 0. 61 mmol) was added and stirred at 50°C for another 0.5 h. Saturated sodium bicarbonate (10 mL) was added and filtered, and the filtrate was concentrated to obtain a crude product, which was isolated by preparative thin-layer chromatography (dichloromethane:methanol=10:1) to obtain trans-*N*-[5-[5-[(4,4-difluoro-1-piperidinyl)methyl]-2-thienyl]-[1,2,4]triazolo[1,5-a]pyridin-2-yl]-2-fluorocyclopropanecarboxamide (EXP-12 ) (5.0 mg, 13% yield, white solid).
**LCMS: $t_R$**= 0.985 min in 5-95AB_1.5min_220&254_Shimadzu.lcm, chromatography (Xtimate C18 2.1*30 mm), MS (ESI) m/z =436.1[M+H]$^+$.
**HPLC:**$t_R$ =2.15 min in 10-80AB_8min_met, chromatography (XBridge Shield RP18 5 um).
**1H NMR(400** MHz, $CD_3OD$) δ = 8.27 (d, *J*=4.0 Hz, 1H), 7.80-7.74 (m, 2H), 7.67-7.64 (m, 1H), 7.47 (d, *J*=3.6 Hz, 1H), 5.01-4.88 (m, 1H), 4.69 (s, 2H), 3.49 (brs, 4H), 2.62-2.42 (m, 1H), 2.38 (brs, 4H), 1.67-1.56 (m, 1H), 1.48-1.44 (m, 1H).
The following embodiment compounds of Table 3 were prepared according to the same method as in the above embodiment, using commercially available compounds or by reference to the preparation method of the intermediate compounds shown.

[Table 3]

| No. | Structure | H-NMR or LC/MS |
|---|---|---|
| EXP-13 | 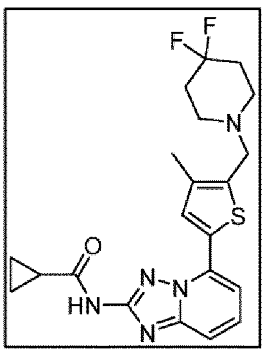 | **LCMS: $t_R$** = 2.264 min in 10-80AB_4min_220&254_Shimadzu.lcm, chromatography (Xtimate C18 2.1 *30 mm), MS (ESI) m/z =432.3[M+H]$^+$. **HPLC: $t_R$** = 3.20 min in 10-80AB_8min_met, chromatography (XBridge Shield RP18, 5 um). **1H NMR**(400 MHz, DMSO-$d_6$): δ = 11.11 (brs, 1H), 8.13 (s, 1H), 7.73-7.56 (m, 3H), 3.77 (s, 2H), 2.62-2.60 (m, 4H), 2.25 (s, 3H), 2.14-2.12 (m, 1H), 1.99-1.95 (m, 4H), 0.88-0.86 (m, 4H). |

Embodiment 6: Preparation of compound EXP-14

A synthetic route is as follows:

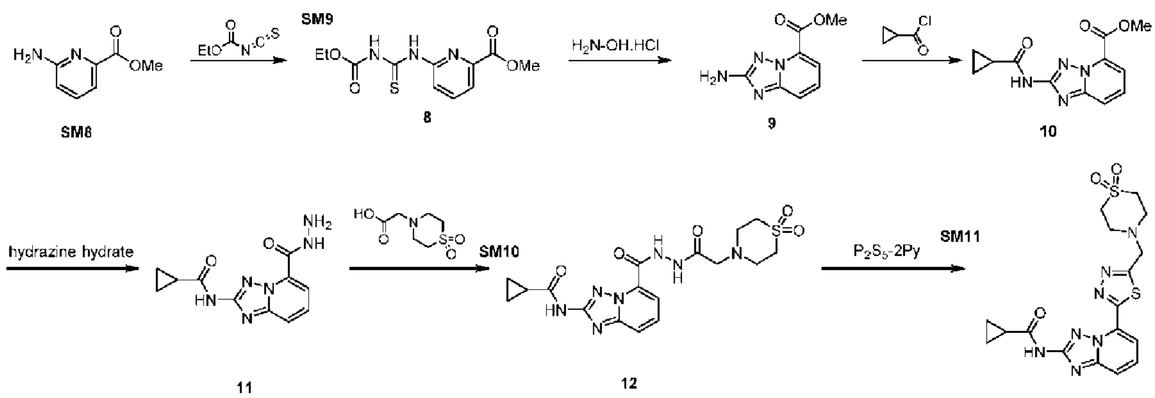


SM8 8 9 10 11 12 EXP-14

Step 6.1 Preparation of compound 8

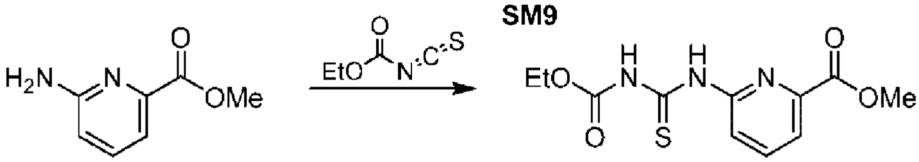


8

SM8

Methyl 6-aminopyridine-2-carboxylate (SM8) (3 g, 19.72 mmol) was dissolved in dioxane (30 mL) and ethoxycarbonyl isothiocyanate (SM9) (2.84 g, 21.69 mmol) was added. The reaction was stirred at 10°C for 16 h. The reaction solution was concentrated and slurried with ethyl acetate/petroleum ether (1/15, 30 mL) to obtain methyl 6-(3-(ethoxy)thiourea)pyridinecarboxylate (8) (5.25 g, 94% yield, 100% purity, pale yellow solid).

**LCMS:$t_R$**= 0.840 min in 5-95AB_220&254_Agilent, chromatography(MERCK RP18e 25*3.0 mm), MS (ESI) m/z =284.1 $[M+H]^+$.

Step 6.2 Preparation of compound 9

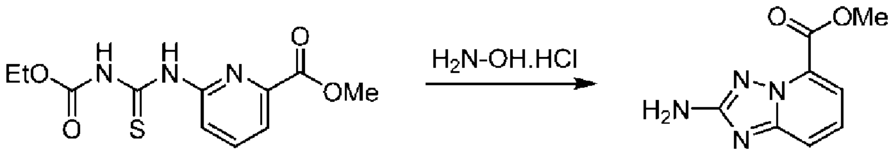


8 9

Methyl 6-(3-(ethoxy)thiourea)pyridinecarboxylate (8) (5.25 g, 18.53 mmol) was dissolved in methanol (75 mL) and hydroxyamine hydrochloride (1.42 g, 20.38 mmol) and diisobutylethylamine (3.11 g, 24.09 mmol) were added. The reaction solution was stirred at 60°C for 16 h. The reaction solution was cooled and filtered. The solid was collected and dried to obtain 2.5 g of white solid product, and the filtrate was concentrated to obtain a crude product, which was then purified by a column (methanol/dichloromethane, 0% to 2%) to obtain 1.5 g of white solid. The two batches of products were combined to obtain methyl 2-amino-[1,2,4]triazolo[1,5-a]pyridine-5-carboxylate (9) (4 g, crude product, white solid).

**1H NMR** (400 MHz,DMSO-$d_6$):δ = 7.62-7.58 (m, 1H), 7.53-7.47 (m, 1H), 7.41 (dd, *J*=7.6, 1.2 Hz, 1H), 6.26 (s, 2H), 3.92 (s, 3H).

Step 6.3 Preparation of compound 10

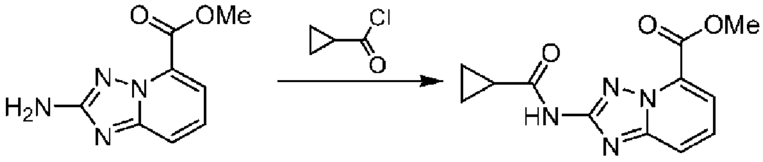

9 10

Methyl 2-amino-[1,2,4]triazolo[1,5-a]pyridine-5-carboxylate (9) (3.56 g, 18.52 mmol) was dissolved in dimethylacetamide (20 mL) and then cyclopropylcarbonyl chloride (SM2) (3.87 g, 37.05 mmol) was added at 0°C. The reaction was stirred at 20°C for 3.5 h. The reaction solution was adjusted to pH = 8 with saturated sodium bicarbonate and filtered. The solid was washed with water (10 mL × 3) and dried to obtain methyl 2-(cyclopropylformylamino)-[1,2,4]triazolo[1,5-a]pyridine-5-carboxylate (10) (2.11 g, 44% yield, 100% purity, white solid).

**LCMS:**$t_R$ = 0.573 min in 5-95AB_1.5min_220&254_ Agilent, chromatography (Merck RP-18e 25-3 mm), MS (ESI) m/z = 261.1 $[M+H]^+$.

Step 6.4 Preparation of compound 11

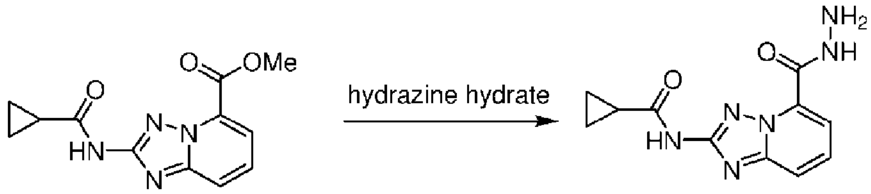


10 11

Methyl 2-(cyclopropylformylamino)-[1,2,4]triazolo[1,5-a]pyridine-5-carboxylate (10) (2.11 g, 8.11 mmol) was dissolved in ethanol (40 mL) and hydrazine hydrate (2.48 g, 48.65 mmol, 98% purity) was added. The reaction was stirred at 13°C for 2 h. The reaction solution was filtrated, and the solid was washed with ethanol (10 mL) and dried to obtain *N*-[5-(hydrazinylformyl)-[1,2,4]triazolo[1,5-a]pyridine-2-]cyclopropanecarboxamide (11) (2 g, 95% yield, 100% purity, white solid). **1H NMR** (400 MHz,DMSO-$d_6$):$\delta$ = 11.45 (br s, 1H), 11.02 (br s, 1H), 7.93-7.86 (m, 1H), 7.85-7.76 (m, 2H), 4.96 (*J*=4.4 Hz, 2H), 2.02 (br s, 1H), 0.94-0.81 (m, 4H).

Step 6.5 Preparation of compound 12

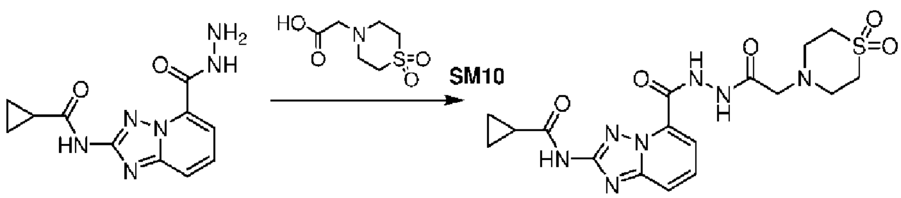


11 12

2-(1,1-dioxo-1,4-thiazin-4-) acetic acid (SM10) (1.63 g, 8.45 mmol) was dissolved in dimethylformamide (20 mL). O-(7-azabenzotriazol-1-)-*N*,*N*,*N*,*N*-tetramethyluronium hexafluorophosphate (3.51 g, 9.22 mmol) and *N*-[5-(hydrazinylformyl)-[1, 2,4]triazolo[1,5-a]pyridine-2-]cyclopropanecarboxamide (11) (2 g, 7.68 mmol) were added sequentially, and finally diisopropylethylamine (1.99 g, 15.37 mmol) was added. The reaction was stirred at 20°C for 3.5 h. The reaction

solution was added to water (100 mL) and filtered. The solid was washed with water (20 mL) and ethanol (40 mL) and dried to obtain *N*-[5-[[[2-(1,1-dioxo-1,4-thiazin-4-)acetyl]amino]carbamoyl]-[1,2,4]triazolo[1,5-a]pyridine-2 -]cyclopropylcarboxamide (12) (3.26 g, 89% yield, 91% purity, white solid).

**LCMS:$t_R$**= 0.546 min in 5-95AB_1.5min_220&254_Shimadzu.lcm, chromatography (Merck RP-18e 25-3 mm), MS (ESI) m/z =436.1 $[M+H]^+$.

Step 6.6 Preparation of compound EXP-14

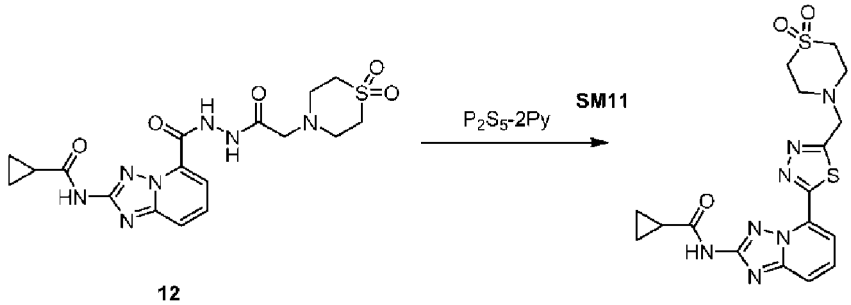


12

EXP-14

*N*-[5-[[[2-(1,1-dioxo-1,4-thiazin-4-)acetyl]amino]carbamoyl]-[1,2,4]triazolo[1,5-a]pyridi ne-2-]cyclopropylcarboxamide (12) (300 mg, 0.689 mmol) was suspended in pyridine (12 mL), and diphosphorus pentasulfide dipyridinium (524 mg, 1.38 mmol) was added. The reaction solution was stirred at 100°C for 16 h. The reaction solution was filtered and the filtrate was concentrated, isolated and purified by preparative HPLC (basic condition: column: YMC-Triart Prep C18 250*50 mm*10 um; mobile phase: [water(0.04% $NH_3H_2O$+10 mM $NH_4HCO_3$)-ACN]; B%: 15% -45%, 8 min) to obtain *N*-[5-[5-[(1,1-dioxo-1,4-thiazin-4-)methyl]-1,3,4-thiadiazol-2-]-[1,2,4]triazolo[1,5-a]pyridine -2-]cyclopropylcarboxamide (EXP-14) (30.4 mg, 10% yield, 97.03% purity, white solid).

**LCMS:**$t_R$ = 1.598 min in 10-80AB_7min_220&254_Shimadzu.lcm, chromatography (Xtimate C18 2.1*30 mm, 3 um), MS (ESI) m/z = 434.1 [M+H]+.

**HPLC : $t_R$** = 2.55 min in 10-80AB_8min.met, chromatography (Ultimate C18 3 um, 3.0*50 mm).

**1H NMR** (400 MHz,DMSO-$d_6$): δ= 11.35 (br s, 1H), 8.22 (dd, *J*=7.2, 0.8 Hz, 1H), 7.96-7.91 (m, 1H), 7.89-7.83 (m, 1H), 4.35 (s, 2H), 3.21-3.13 (m, 4H), 3.13-3.05 (m, 4H), 2.08 (br s, 1H), 0.95-0.84 (m, 4H).

Embodiment 7: Preparation of compound EXP-15

A synthetic route is as follows:

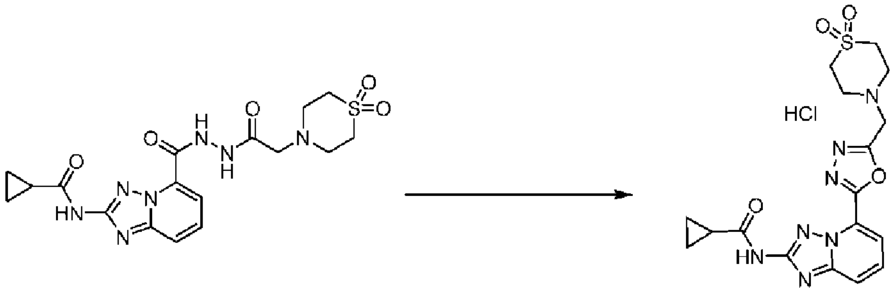


12

EXP-15

Step 7.1 Preparation of compound EXP-15

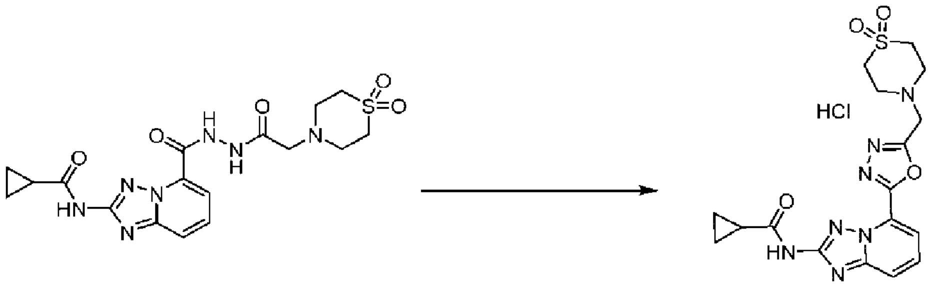


12 EXP-15

*N*-[5-[5-[(1,1-dioxo-1,4-thiazin-4-)methyl]-1,3,4-thiadiazol-2-]-[1,2,4]triazolo[1,5-a]pyri dine-2-]cyclopropylcarboxamide (12) (180 mg, 0.413 mmol) was dissolved in thionyl chloride (3 mL) and dimethylformamide (0.3 mL). The reaction was stirred at 20°C for 4 h. The reaction solution was concentrated and then adjusted to pH = 8 by adding saturated sodium bicarbonate, and was extracted with dichloromethane (40 mL × 3). The organic phases were combined and saline solution (10 mL) was added. The residue was dried over sodium sulfate, filtered, concentrated, isolated and purified by preparative HPLC (HCl condition: column: ACE 5 C18-AR 150*30 mm*5 μm; mobile phase: [water (0.05%HCl)-ACN]; B%: 10%-40%, 8 min) to obtain *N*-[5-[5-[(1,1-dioxo-1,4-thiazin-4-)methyl]-1,3,4-oxadiazole-2-]-[1,2,4]triazolo[1,5-a]pyridine-2-]cyclopropylcarboxamide (EXP-15) (27.1 mg, 14.40% yield, 99.73% purity, hydrochloride, yellow solid).

LCMS: $t_R$= 2.341 min in 0-60AB_7min_220&254_Shimadzu.lcm, chromatography (Xtimate C18 2.1*30 mm, 3 um), MS (ESI) m/z = 418.2 $[M+H]^+$.

HPLC: $t_R$ =3.12 min in 0-60AB_8min.met, chromatography (Ultimate C18 3 um, 3.0*50 mm).

**1H NMR** (400 MHz,DMSO-$d_6$):δ= 11.28 (br s, 1H), 8.00-7.99 (m, 1H), 7.87-7.82 (m, 2H), 4.31 (s, 2H), 3.22 (s, 8H), 2.07 (br s, 1H), 0.93-0.77 (m, 4H).

Embodiment 8: Preparation of compound EXP-16

A synthetic route is as follows:

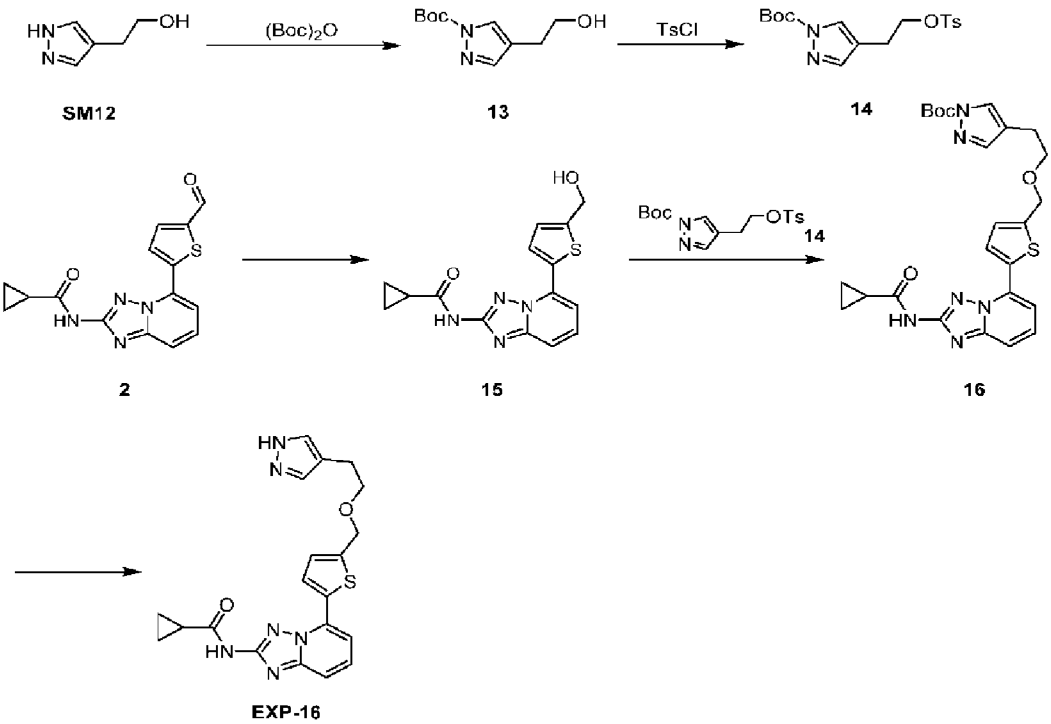


SM12 13 14

2 15 16

EXP-16

Step 8.1 Preparation of compound 13

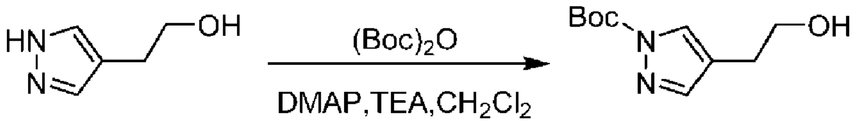


**SM12** **13**

2-(1*H*-pyrazol-4-yl)ethanol (SM12) (200 mg, 1.78 mmol), triethylamine (361 mg, 3.57 mmol), dimethylaminopyridine (44 mg, 0.36 mmol) and Boc anhydride (467 mg, 2.14 mmol) were dissolved in dichloromethane (3 mL) and stirred at 0-20°C for 12 h under nitrogen protection. The reaction solution was concentrated to obtain tert-butyl 4-(2-hydroxyethyl)pyrazole-1-carboxylate (13) (110 mg, crude product) as yellow oil, which was used directly in the next step.

Step 8.2 Preparation of compound 14

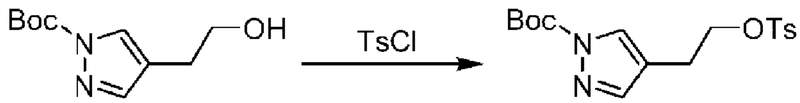


**13** **14**

Tert-butyl 4-(2-hydroxyethyl)pyrazole-1-carboxylate (13) (100 mg, 0.47 mmol), p-toluenesulfonyl chloride (99 mg, 0.52 mmol) and triethylamine (95 mg, 0.94 mmol) were dissolved in dichloromethane (3 mL) and stirred at 0-20°C for 2 h under nitrogen protection. The reaction solution was concentrated to obtain 4-[2-(p-toluenesulfonyloxy)ethyl]pyrazole (14) (120 mg, crude product) as yellow oil, which was used directly in the next step.

**LCMS:** $t_R$= 2.850 min in 10-80AB_4min_220&254_Shimadzu, chromatography (Xtimate C18, 3 um, 2.1*30 mm), MS (ESI) m/z =367.2[M+H]$^+$.

**1H NMR** (400 MHz, DMSO-$d_6$): δ = 8.05 - 7.98 (m, 1H), 7.74 (d, *J*= 8.0 Hz, 2H), 7.62 (s, 1H), 7.45 (d, *J* = 8.0 Hz, 2H), 4.20 (t, *J* = 6.4 Hz, 2H), 2.77 (t, *J* = 6.4 Hz, 2H), 2.42 (s, 3H), 1.57 (s, 9H).

Step 8.3 Preparation of compound 15

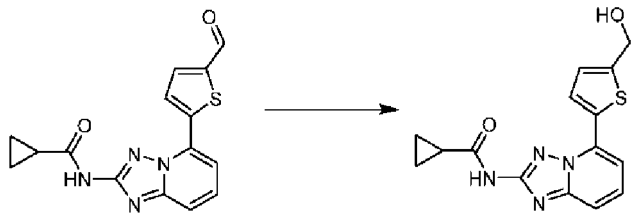

**2** **15**

*N*-[5-(5-formyl-2-thienyl)-[1,2,4]triazolo[1,5-a]pyridin-2-yl]cyclopropanecarboxamide (2) (30 mg, 0.1 mmol) was dissolved in methanol (2 ml) and then sodium cyanoborohydride (12 mg, 0.19 mmol) was added. The reaction was stirred at 50°C for 0.2 h under nitrogen protection. Then sodium bicarbonate (1 mL) was added and the mixture was extracted with ethyl acetate (10 mL × 3). The organic phases were combined, washed with saturated saline solution (5 ml), dried over anhydrous sodium sulfate, filtered and concentrated to obtain *N*-[5-[5-(hydroxymethyl)-2-thienyl]-[1,2,4]triazolo[1,5-a]pyridin-2-yl]cyclopropanecarboxa mide (15) (30 mg, crude product) as a yellow solid.

**LCMS:** $t_R$= 2.099 min in 10-80AB_4.0 min_220&254_Shimadzu.lcm, chromatography (Xtimate C18, 3 um, 2.1*30 mm), MS (ESI) m/z =315.1 [M+H]$^+$.

**1H NMR** (400 MHz, DMSO-$d_6$): δ = 11.14 (s, 1H), 8.15 (d,*J* = 3.6 Hz, 1H), 7.60-7.52 (m, 3H), 7.03 (d, *J* = 3.6 Hz, 1H), 5.58 (t, *J* = 5.6 Hz, 1H), 4.64 (d, *J* = 5.6 Hz, 2H), 2.01-1.98 (m, 1H), 0.81-0.76 (m, 4H).

Step 8.4 Preparation of compound 16

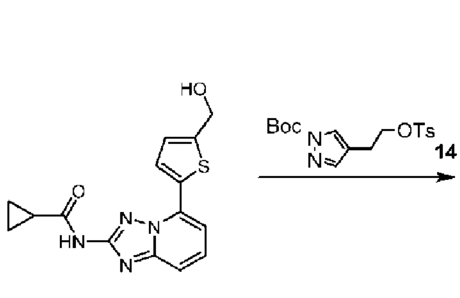


15 16

Tert-butyl 4-[2-(p-toluenesulfonyloxy)ethyl]pyrazole-1-carboxylate (14) (23 mg, 0.06 mmol), N-[5-[5-(hydroxymethyl)-2-thienyl]-[1,2,4]triazolo[1,5-a]pyridin-2-yl]cyclopropanecarboxa mide (15) (20 mg, 0.06 mmol), and cesium carbonate (21 mg, 0.06 mmol) were dissolved in dimethyl sulfoxide (2 mL). The reaction was replaced with nitrogen for three times, and stirred at 60°C for 0.5 h. Water (6 mL) was added and the mixture was extracted with ethyl acetate (10 mL × 3). The organic phases were combined, washed with saturated saline solution (5 mL), dried over anhydrous sodium sulfate, filtered, and concentrated to obtain *N*-[5-[5-(4-(2-hydroxyethyl)pyrazole-1-carboxylic acid tert-butyl ester-hydroxymethyl)-2-thienyl]-[1,2,4]triazolo[1,5-a]pyridin-2-yl]cyclopropanecarboxamide (16) (27 mg, crude product) as a yellow solid.

**LCMS:** $t_R$= 1.156 min in 5-95AB_1.5min_220&254_Shimadzu.lcm, chromatography (Xtimate C18, 3 um, 2.1*30 mm), MS (ESI) m/z =509.2[M+H]$^+$.

Step 8.5 Preparation of compound EXP-16

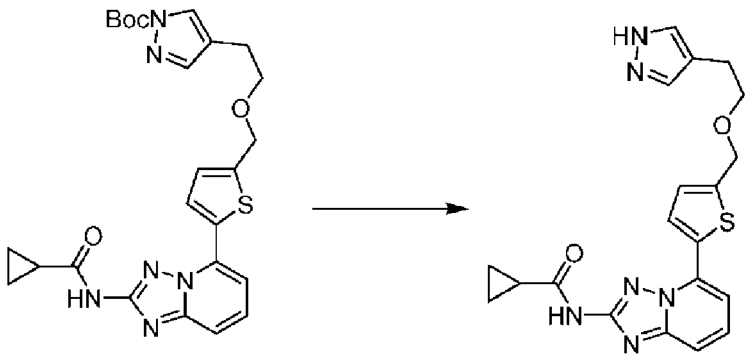

16 EXP-16

*N*-[5-[5-(4-(2-hydroxyethyl)pyrazole-1-carboxylic acid tert-butyl ester-hydroxymethyl)-2-thienyl]-[l,2,4]triazolo[1,5-a]pyridin-2-yl]cyclopropanecarboxamide (16) (27 mg, 0.05 mmol) was dissolved in dichloromethane (2 mL) and trifluoroacetic acid (29 mg, 0.256 mmol) was added at room temperature. The reaction was stirred at room temperature under nitrogen protection for 0.5 h. The reaction solution was concentrated to obtain a crude product, which was purified by preparative thin layer chromatography (dichloromethane:methanol=10:1) to obtain the title product *N*-[5-[5-[2-(1*H*-pyrazol-4-yl)-ethoxymethyl]-2-thienyl]-[1,2,4]triazolo[1,5-a]pyridin-2-yl]cyc lopropanecarboxamide (EXP-16) (3.0 mg, 14% yield, white solid).

**LCMS:** $t_R$= 1.872 min in 10-80AB_4min_220&254_Shimadzu.lcm, chromatography (Xtimate C18 2.1*30 mm), MS (ESI) m/z =409.3 [M+H]$^+$.

**HPLC:** $t_R$ = 2.39 min in 10-80CD_8min_met, chromatography (XBridge Shield RP18 5 um).

**1H NMR** (400MHz, $CD_3OD$): $\delta$ = 8.13 (d, *J*= 4.0 Hz, 1H), 7.80-7.70 (m, 2H), 7.63 (dd, *J* = 8.4 and 1.2 Hz, 1H), 7.49 (s, 2H), 7.16 (d, *J* = 3.6 Hz, 1H), 4.86-4.84 (m, 2H), 4.24-4.20 (m, 2H), 2.93-2.89 (m, 2H), 2.53-2.47 (m, 1H), 1.06-1.03 (m, 2H), 0.90-0.89 (m, 2H).

Embodiment 9: Preparation of compound EXP-17

A synthetic route is as follows:

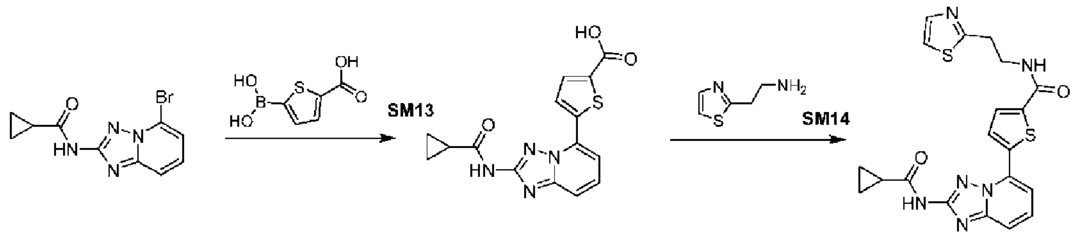


Int-1 17 EXP-17

Step 9.1 Preparation of compound 17

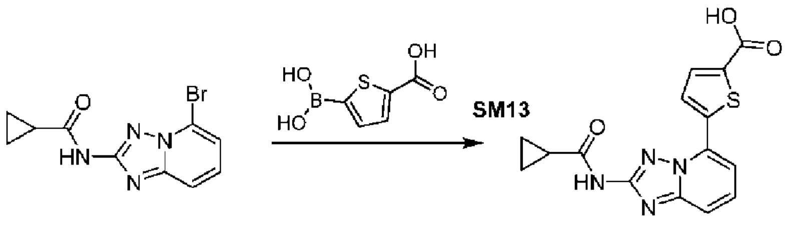


Int-1 17

*N*-(5-bromo-[1,2,4]triazolo[1,5-a]pyridin-2-yl)-cyclopropanecarboxamide (Int-1) (100 mg, 0.36 mmol), 5-carboxy-2-thiopheneboronic acid (SM13) (122 mg, 0.71 mmol) and sodium carbonate (113 mg, 1.07 mmol) were dissolved in tetrahydrofuran (10 mL) and water (5 mL). After replacing with nitrogen for three times, 1,1-bis(diphenylphosphine)ferrocene palladium chloride (50 mg, 0.07 mmol) was added. After replacing with nitrogen for three times, the reaction was raised to 100°C and stirred for 2 h. The reaction solution was added with water (10 mL) and extracted with ethyl acetate (20 mL). The organic phase was washed with saturated saline solution (10 mL), dried over anhydrous sodium sulfate and then filtered. The filtrate was concentrated under vacuum to obtain 5-(2-formyl)-thiophene-[1,2,4]triazolo[1,5-a]pyridine-2-cyclopropanecarboxamide (17) (50 mg, crude product) as a yellow solid, which was used directly in the next step.

**LCMS:$t_R$**= 0.783 min in 5-95AB_1.5min_220&254_Shimadzu.lcm, chromatography (Xtimate C18, 3 um, 2.1*30 mm), MS (ESI) m/z = 329.0 $[M+H]^+$.

Step 9.2 Preparation of compound EXP-17

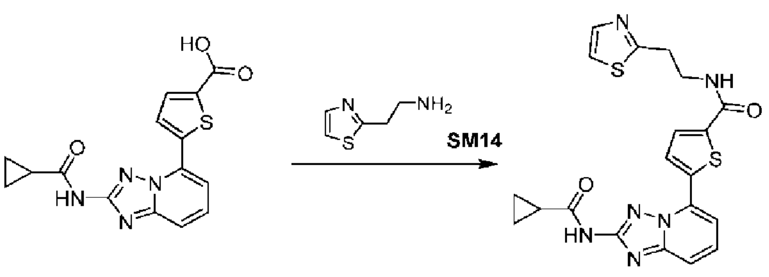


17 EXP-17

5-(2-carboxy)-thiophene-[1,2,4]triazolo[1,5-a]pyridine-2-cyclopropanecarboxamide (17) (50 mg, 0.15 mmol) and 2-thiazol- 2-ylethylamine hydrochloride (SM14) (46 mg, 0.23 mmol) were dissolved in dimethylformamide (2 mL) and O-(7-azabenzotriazol-1-yl)-*N*,*N*,*N*,*N*-tetramethyluronium hexafluorophosphate (75 mg, 0.20mmol) and triethylamine

(46 mg, 0.46 mol) were added. The reaction solution was stirred at 25°C for 0.2 h.

The reaction solution was concentrated and then purified by preparative HPLC [Column: Phenomenex Gemini-NX 150*30 mm*5 um; Condition: 25-55%B (A: water (0.04%$NH_3.H_2O$+10mM $NH_4HCO_3$); B: $CH_3CN$); Flow rate: 25 mL/min] to obtain 5-[2-[2-thiazole-2-ethyl]carboxamido]-thiophene-[1,2,4]triazolo[1,5-a]pyridine-2-cyclopropa necarboxamide (EXP-17) (6.3 mg, 8% yield, white solid).

**LCMS:$t_R$**= 2.468 min in 10-80AB_4min_220&254_Shimadzu.lcm, chromatography (Xtimate C18 2.1*30 mm), MS (ESI) m/z =439.2[M+H]$^+$.

**HPLC: $t_R$** = 3.51 min in 50-100AB_8min_met, chromatography (XBridge Shield RP18 5 um).

**1H NMR** (400 MHz, DMSO-$d_6$):δ = 11.21 (brs, 1H), 8.90 (t, *J*= 4.8 Hz, 1H), 8.32 (d, *J*=4.0 Hz, 1H), 7.87-7.81 (m, 2H), 7.78-7.68 (m, 3H), 7.61 (d, *J*=3.2 Hz, 1H), 3.65 (dd, *J*= 12.8 and 7.2 Hz, 2H), 2.58-2.54 (m, 2H), 2.14-2.12 (m, 1H), 0.92 - 0.86 (m, 4H).

The following embodiment compound of Table 4 was prepared according to the same method as in the above embodiment, using commercially available compounds or by reference to the preparation method of the intermediate compounds shown.

[Table 4]

| No. | Structure | H-NMR or LC/MS |
|---|---|---|
| EXP-18 | 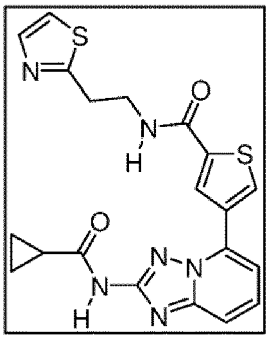 | **LCMS:$t_R$**= 3.281 min in 10-80AB_7min_220&254_Shimadzu.lcm (Xtimate C18, 3 um, 2.1*3 mm), MS (ESI) m/z =439.3 [M+H]$^+$. **HPLC:$t_R$**= 2.28 min in 10-80CD 8min.met, chromatography (XBridge Shield RP18 5 um). **1H NMR** (400 MHz,DMSO-$d_6$):δ =11.18 (s, 1H), 9.15 (s, 1H), 8.83 (t, *J*=5.6 Hz, 1H), 8.52 (d, *J*=1.2 Hz, 1H), 7.79-7.72 (m, 2H), 7.71-7.66 (m, 1H), 7.59 (d, J=3.2Hz, 1H), 7.51 (dd, *J*= 7.2, 1.2 Hz, 1H), 3.71-3.59 (m, 2H), 3.29-3.26 (m, 2H), 2.05-1.94 (m, 1H), 0.89-0.82 (m, 4H). |

Embodiment 10: Preparation of compound EXP-19

A synthetic route is as follows:

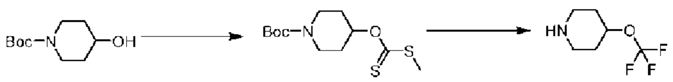

SM15 18 19

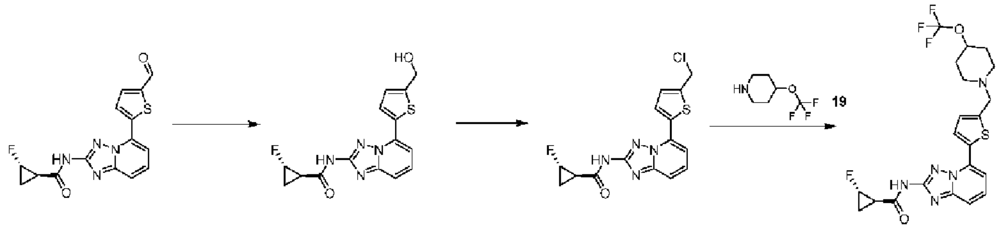


7 20 21 EXP-19

Step 10.1 Preparation of compound 18

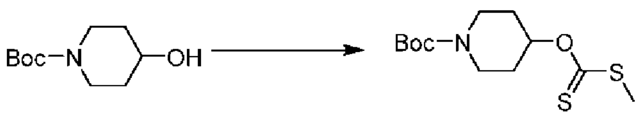

SM15 18

4-hydroxy-*N*-Boc-piperidine (SM15) (2 g, 9.94 mmol) was dissolved in tetrahydrofuran (20 mL), and sodium hydride (1.19 g, 29.81 mmol, 60% purity) was added in portions at 0°C. After stirring for 30 min, carbon disulfide (3.03 g, 39.75 mmol, 2.40 mL) was added and stirring was continued for 30 min. Iodomethane (2.82 g, 19.87 mmol) was added and then reacted at room temperature for 1 h. The reaction solution was poured into water (20 mL). The aqueous phase was extracted with ethyl acetate (20 mL × 3), and the organic phase was dried over anhydrous sodium sulfate, filtered, concentrated and spin-dried, and then subjected to silica gel column chromatography (eluent: 0 %-10% ethyl acetate/petroleum ether) to obtain the pale yellow target product *N*-tert-butoxycarbonylpiperidine-4-methylxanthate (18) (1.6 g, yield 55%).

**1H NMR** (400 MHz, DMSO-$d_6$): δ = 5.74-5.60 (m, 1H), 3.62-3.48 (m, 2H), 3.32-3.23 (m, 2H), 2.55 (s, 3H), 2.02-1.86 (m, 2H), 1.71-1.63 (m, 2H), 1.40 (s, 9H).

Step 10.2 Preparation of compound 19

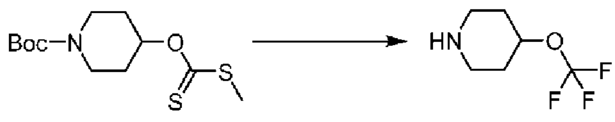

18 19

1,3-dibromo-5,5-dimethylhydantoin (2.21 g, 7.72 mmol) was dissolved in dichloromethane (20 mL), and a solution of hydrogen fluoride-pyridine (7.14 g, 72.06 mmol) and a solution of *N*-tert-butoxycarbonyl piperidine-4 methylxanthate (18) (500 mg, 1.72 mmol) in dichloromethane (2 mL) were added in portions at -75°C under nitrogen atmosphere. The reaction solution was reacted at 15-20°C for 16 h and then quenched with saturated sodium bicarbonate solution (20 mL), and the aqueous phase was extracted with ethyl acetate (20 mL × 3). The organic phase was dried over anhydrous sodium sulfate, filtered, concentrated and spin-dried to obtain the yellow target product 4-(trifluoromethoxy)piperidine (19) (400 mg, crude product).

**1H NMR** (400 MHz, DMSO-$d_6$): δ = 8.79 (s, 1H), 4.81-4.52 (m, 1H), 3.30-3.17 (m, 2H), 3.16-3.03 (m, 2H), 2.16-1.99 (m, 2H), 1.94-1.72 (m, 2H).

Step 10.3 Preparation of compound 20

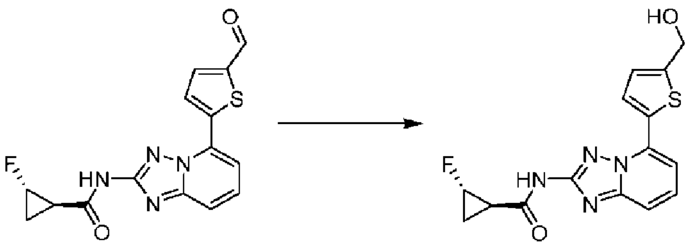

7 20

Trans-2-fluoro-*N*-[5-(5-formyl-2-thiophene)-[1,2,4]triazolo[1,5-a]pyridin-2-yl]cycloprop anecarboxamide (7) (180 mg, 0.54 mmol) was dissolved in tetrahydrofuran (3 mL), and lithium aluminum hydride (41 mg, 1.09 mmol) was added at 0°C. The mixed reaction solution was reacted at 15-20°C for 2 h and then quenched with 1 M HCl solution (20 mL), and the aqueous phase was extracted with dichloromethane (20 mL × 3). The organic phase was dried over anhydrous sodium sulfate, filtered, concentrated and spin-dried to obtain the dark yellow target product trans-2-fluoro-*N*-[5-(5-hydroxymethyl-2-thiophene)-[1,2,4]triazolo[1,5-a]pyridin-2-yl]cyclop ropanecarboxamide (20) (80 mg, yield 44%).

**1H NMR** (400 MHz, DMSO-$d_6$): δ=11.33 (s, 1H), 8.26 (d, *J* = 4.0 Hz, 1H), 7.74-7.60 (m, 3H), 7.13 (d, *J* = 4.0 Hz, 1H), 5.10-4.83 (m, 1H), 4.74 (s, 2H), 2.42-2.36 (m, 1H), 1.65-1.50 (m, 1H), 1.37-1.27 (m, 1H).

Step 10.4 Preparation of compound 21

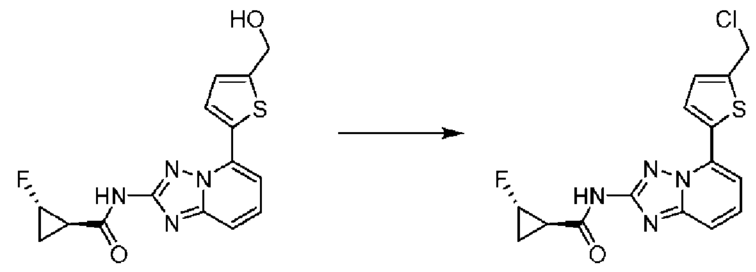

20 21

Trans-2-fluoro-*N*-[5-(5-hydroxymethyl-2-thiophene)-[1,2,4]triazolo[1,5-a]pyridin-2-yl]c yclopropanecarboxamide (20) (80 mg, 0.24 mmol) was dissolved in dichloromethane (2 mL), and thionyl chloride (286 mg, 2.41 mmol) was added thereto at 0°C. The mixed reaction solution was reacted at 15-20°C for 16 h and then concentrated and spin-dried to obtain the brown target product trans-2-fluoro-*N*-[5-(5-chloromethyl-2-thiophene)-[1,2,4]triazolo[1,5-a]pyridin-2-yl]cyclopro panecarboxamide (21) (60 mg, yield 71%).

Step 10.5 Preparation of compound EXP-19

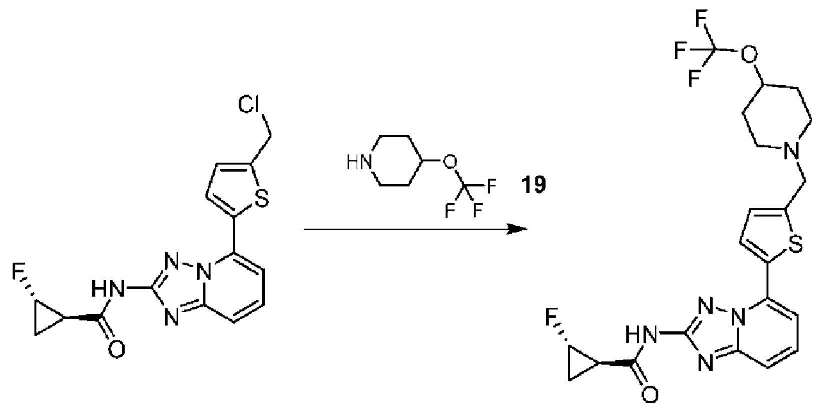


21 EXP-19

Trans-2-fluoro-N-[5-(5-chloromethyl-2-thiophene)-[1,2,4]triazolo[1,5-a]pyridin-2-yl]cyc lopropanecarboxamide (21) (60 mg, 0.17 mmol) was dissolved in N,N-dimethylformamide (2 mL), and potassium carbonate (70 mg, 0.51 mmol) and 4-(trifluoromethoxy)piperidine (19) (58 mg, 0.34 mmol) were added thereto. The reaction solution was reacted at 15-25°C for 2 h. The reaction mixture was diluted with dichloromethane (20 mL) and water (20 mL). The aqueous phase was extracted with dichloromethane (20 mL × 3), the organic phase was washed with saturated saline solution (20 mL × 3), dried over anhydrous sodium sulfate, filtered, concentrated and spin-dried, and then purified by HPLC (separation column: Waters Xbridge 150*25 mm* 5 um; mobile phase: A: water (0.04% $NH_3.H_2O$+10 mM $NH_4HCO_3$) and B (acetonitrile; gradient: B 50-80 %) to obtain the white solid trans 2-fluoro-*N*-[5-[5-[5-[[4-(trifluoromethyl)-1-piperidine]methoxy]-2-thiophene]-[1,2,4]triazolo[ 1,5-a]pyridin-2-yl]cyclopropanecarboxamide (EXP-19) (1.8 mg, yield 2%, purity 98.56%).

**LCMS:** $t_R$ = 1.773 min in 10-80AB_4min_220&254_Shimadzu.lcm (Xtimate C18, 3 um, 2.1 *3 mm), MS (ESI) m/z = 484.30 $[M+H]^+$.

**HPLC:**$t_R$ = 2.58 min in 10-80AB_8min.met, chromatography.

**1H NMR** (400 MHz, DMSO-$d_6$): δ = 11.31 (s, 1H), 8.25 (d, *J*= 4.0 Hz, 1H), 7.80-7.55 (m, 3H), 7.15 (d, *J* = 4.0 Hz, 1H), 5.06-4.83 (m, 1H), 4.53-4.41 (m, 1H), 3.79 (s, 2H), 2.79-2.71 (m, 2H), 2.43-2.41 (m, 1H), 2.39-2.33 (m, 2H), 1.97-1.87 (m, 2H), 1.80-1.67 (m, 2H), 1.65-1.51 (m, 1H), 1.40-1.27 (m, 1H).

Embodiment 11: Detection of JAK kinase inhibitory activity

The compounds of the above embodiments were applied to the detection and screening of JAK kinase inhibitory activity.

1. Method

A corresponding volume of DMSO was added to each well of a first 96-well plate. 20-30 μL of 100-200 μM stock solution of different test compounds were added to each well. The mixture was shaken and mixed for 3 min. All of the compounds were subjected to 3X serial dilution. 60-80 μL Kinase buffer was added to each well of a second 96-well plate, and 1-2 μL of the solution taken from each well of the first 96-well plate was added to the corresponding well of the second 96-well plate. The mixture was shaken and mixed for 3 min. 5 μL of compound dilution taken from each well of the second compound dilution plate was transferred to the corresponding well of a 384-well test plate. 1-2μE TK Substrate-biotin was added to each well of the test plate. 1-2 μL of enzyme mixture was added to each well. At the same time, a blank well without enzyme was set. 1-2 μL of ATP solution was added to each well, the plate was sealed and reacted at room temperature. The reaction time was as follows: JAK1: 2 h, JAK2: 30 min, JAK3: 30 min, and TYK2: 50 min. 3-5 μL of Streptavidin-XL665 and 3-5 μL of TK Antibody-cryptate were added to each well, the plate was sealed and allowed to stand at room temperature for 30 min to complete the reaction. The fluorescence at 665 nm and 620 nm were read on PerkinElmer EnVision instrument.

IC50 calculation

The HTRF ratio of each well was calculated as: (665 signal/620 signal) x 10^4.

The percentage of inhibition was calculated based on the following formula:

$$\text{Inhibition } \% = [(\text{Ratio}_{max}-\text{Ratio}_{compound})/(\text{Ratio}_{max}-\text{Ratio}_{min})]\times 100\%$$

$\text{Ratio}_{compound}$ is the HTRF ratio at a given compound concentration, $\text{Ratio}_{min}$ is the HTRF ratio added to the blank well, and $\text{Ratio}_{max}$ is the HTRF ratio without adding the compound. GraphPad Prism 5.0 software was used to plot a sigmoid dose-inhibition rate curve with a non-linear regression model and calculate the $IC_{50}$ value.

Filgotinib represents a compound with the following structure:

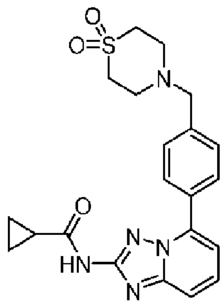

Filgotinib

2. Test result

The $IC_{50}$ (nM) values for each test compound are shown in Table 5 below.

[Table 5] Inhibitory activity of compounds on JAK

| Code ID | Biochemical assay $IC_{50}$ (nM) | | | |
|---|---|---|---|---|
| | JAK1 | JAK2 | JAK3 | TYK2 |
| EXP-1 | C | C | D | E |
| EXP-2 | D | D | NT | NT |

(continued)

| Code ID | Biochemical assay $IC_{50}$ (nM) | | | |
|---|---|---|---|---|
| | JAK1 | JAK2 | JAK3 | TYK2 |
| EXP-3 | B | **C** | E | E |
| EXP-4 | E | NT | NT | NT |
| EXP-5 | D | D | NT | NT |
| EXP-6 | D | D | E | E |
| EXP-7 | E | E | NT | NT |
| EXP-8 | E | E | NT | NT |
| EXP-9 | D | D | NT | NT |
| EXP-10 | E | NT | NT | NT |
| EXP-11 | D | D | NT | NT |
| EXP-12 | D | NT | NT | NT |
| EXP-13 | **C** | **c** | NT | NT |
| EXP-14 | E | E | NT | NT |
| EXP-15 | E | NT | NT | NT |
| EXP-16 | E | NT | NT | NT |
| EXP-17 | E | NT | NT | NT |
| EXP-18 | D | D | NT | NT |
| EXP-19 | E | NT | NT | NT |
| Filgotinib | **C** | D | E | E |
| Note: $IC_{50}$ value between 0-10 nM is marked as A; 10-30 nM is marked as B; 30-50 nM is marked as C; 50-500 nM is marked as D; greater than 500 nM is marked as E; and NT stands for untested. | | | | |

Embodiment 12: Detection of cell proliferation inhibition

Embodiment 12-1: Detection of proliferation inhibition based on Hela cells

Method:

1. Cell plating: cultured cells were collected and counted for viable cells with Vi-Cell XR cell counter. The cell suspension was adjusted to an appropriate concentration with the medium and then added to a 96-well cell culture plate. 50 μL-100 μL of the cell suspension was added to each well of the 96-well cell culture plate. The cell plating density depended on the growth rate of the cells. The cells were cultured in corresponding incubators.
2. Drug treatment and OSM stimulation
Firstly, a series of test compound concentration (work solution-1) was prepared using DMSO with 3X gradient dilution, and then it was subjected to fold dilution with RPMI1640+10% FBS to obtain a work solution-2; Cisplatin's work solution-2 was prepared with RPMI1640+10% FBS, and then 1-10 μL solution taken from the work solution-2 was added to the prepared cell wells containing medium. After incubation, 1-10 μL of OSM medium was added to each well and continued to culture. An appropriate CTG solution was added to each well. The mixture was shaken and mixed for 1-5 min and then a luminescence signal was determined with an Envision2104 plate reader.

Cell survial rate calculation

$$\text{Formula: } T/C \times 100\%$$

T is the luminescence reading of a drug treatment group, and C is the average luminescence reading of a solvent control group. GraphPad Prism 5.0 software was used to plot a sigmoid dose-inhibition rate curve with a non-linear regression model and calculate the IC50 (T/C × 100%=50%) value.

Test result

The $IC_{50}$ (nM) values of each test compound are shown in Table 6 below.

[Table 6] Detection of compound's proliferation inhibitory activity on Hela cells

| Code ID | $IC_{50}$ (uM) |
|---|---|
| EXP-1 | A |
| EXP-2 | A |
| EXP-3 | A |
| EXP-4 | B |
| EXP-5 | B |
| EXP-6 | NT |
| EXP-7 | NT |
| EXP-8 | NT |
| EXP-9 | NT |
| EXP-10 | NT |
| EXP-11 | NT |
| EXP-12 | NT |
| EXP-18 | NT |
| EXP-19 | NT |
| Filgotinib | B |
| Note: $IC_{50}$ value from 0-5 uM is marked as A; 5-30 uM is marked as B; and NT stands for untested. | |

Embodiment 12-2: Detection of proliferation inhibition based on BaF3 cells

Method:

1. Cell plating: cultured cells were collected with RPMI1640+10% FBS (containing IL-3) medium and counted for viable cells with Vi-Cell XR cell counter. The cell suspension was adjusted to an appropriate concentration with RPMI1640+10% FBS (containing IL-3) medium and then added to a 96-well cell culture plate. A certain amount of cell suspension was added to each well of the 96-well cell culture plate. The cells were cultured in corresponding incubators.
2. Drug treatment: a series of test compound concentration (work solution-1) was prepared using DMSO with 3X gradient dilution, and then it was subjected to fold dilution with RPMI1640+10% FBS (containing IL-3) to obtain a work solution-2; Cisplatin's work solution-2 was prepared with RPMI1640+10% FBS (containing IL-3), and then 1-10 μL solution taken from the work solution-2 was added to the prepared cell wells containing medium. The cells were cultured in the corresponding incubators. An appropriate CTG solution was added to each well. The mixture was shaken and mixed for 5-15 min and then a luminescence signal was determined with an Envision2104 plate reader.

Cell survial rate calculation

$$\text{Formula: } T/C \times 100\%$$

T is the luminescence reading of a drug treatment group, and C is the average luminescence reading of a solvent control group. GraphPad Prism 5.0 software was used to plot a sigmoid dose-inhibition rate curve with a non-linear regression model and calculate the IC50 (T/C × 100%=50%) value.

[Table 7] Detection of compound's proliferation inhibitory activity on BaF3 cells

| Code ID | $IC_{50}$ (uM) |
|---|---|
| EXP-1 | A |
| EXP-2 | A |
| EXP-3 | A |
| EXP-4 | B |
| EXP-5 | A |
| EXP-6 | NT |
| EXP-7 | NT |
| EXP-8 | NT |
| EXP-9 | NT |
| EXP-10 | NT |
| EXP-11 | NT |
| EXP-12 | NT |
| EXP-18 | NT |
| EXP-19 | NT |
| Filgotinib | B |
| Note: $IC_{50}$ value from 0-5 uM is marked as A; 5-30 uM is marked as B; and NT stands for untested. | |

Embodiment 12-3: Detection of proliferation inhibition based on THP1 cells

Method:

1. Cell plating: cultured cells were collected and counted for viable cells with Vi-Cell XR cell counter. The cell suspension was adjusted to an appropriate concentration with the medium and then added to a 96-well cell culture plate. 80 μL of the cell suspension was added to each well of the 96-well cell culture plate. The cell plating density depended on the growth rate of the cells. The cells were cultured in corresponding incubators.
2. Drug treatment and IL-4 stimulation: firstly, a series of test compound concentration (work solution-1) was prepared using DMSO with 3X gradient dilution, and then it was subjected to fold dilution with RPMI1640+10% FBS to obtain a work solution-2; Cisplatin's work solution-2 was prepared with RPMI1640+10% FBS, and then 1-10 μL solution taken from the work solution-2 was added to the prepared cell wells containing medium. The cells were cultured in the corresponding incubators. After incubation, 1-10 μL of IL-4 medium was added to each well and continued to culture. An appropriate CTG solution was added to each well. The mixture was shaken and mixed for 5-15 min and then a luminescence signal was determined with an Envision2104 plate reader.

Cell survial rate calculation

$$\text{Formula: } T/C \times 100\%$$

T is the luminescence reading of a drug treatment group, and C is the average luminescence reading of a solvent control group. GraphPad Prism 5.0 software was used to plot a sigmoid dose-inhibition rate curve with a non-linear regression model and calculate the IC50 (T/C x 100%=50%) value.

[Table 8] Detection of compound's proliferation inhibitory activity on THP1 cells

| Code ID | $IC_{50}$ (uM) |
|---|---|
| EXP-1 | A |

(continued)

| Code ID | $IC_{50}$ (uM) |
|---|---|
| EXP-2 | A |
| EXP-3 | A |
| EXP-4 | B |
| EXP-5 | A |
| EXP-6 | NT |
| EXP-7 | NT |
| EXP-8 | NT |
| EXP-9 | NT |
| EXP-10 | NT |
| EXP-11 | NT |
| EXP-12 | NT |
| EXP-18 | NT |
| EXP-19 | NT |
| Filgotinib | B |
| Note: $IC_{50}$ value from 0-5 uM is marked as A; 5-30 uM is marked as B; and NT stands for untested. | |

Embodiment 13: Stability of liver microsomes

Liver microsomes belonging to different species (human, rat) were respectively reacted with the compound according to the present invention for a certain period of time, and the residual rate was calculated by comparing a reacted sample with an unreacted sample, and the extent to which the compounds according to the present invention were metabolized by the liver was evaluated.

(Result) shows the residual rate of the compound in oxidative metabolism at 10 umol/L. Compound EXP-3: human liver microsomes: 59.32%; rat liver microsomes: 55.3%.

Embodiment 14: CYP inhibition test

Commercially available mixed human liver microsomes were used for the typical substrate metabolism reaction of the main human CYP molecular species (CYP1A2, 2B6, 2C8, 2C9, 2C19, 2D6 and 3A4). The reaction conditions were as follows: substrate, CYP1A2: 30 μM of phenacetin; CYP2C9: diclofenac 10 μM; CYP2C19: 35 μM of S-meth-phenytoin; CYP3A4: midazolam 5 μM, testosterone 80 μM; CYP2D6: bufuralol, 10 μM; CYP2C8: 10 μM of paclitaxel; and CYP2B6: bupropion 70 μM; reaction time, CYP1A2, 2C9, 2D6, 2C8, 2B6: 10 min; CYP2C19: 45 min; and CYP3A4: 5 min. Reaction temperature, 37°C. Inhibitor (positive control), CYP1A2: α-naphthoflavone; CYP2C9: sulfaphenazole; CYP2C19: omeprazole; CYP3A4: ketoconazole; CYP2D6: quinidine; CYP2C8: nicardipine; and CYP2B6: clopidogrel.

A series of dilutions for the test compound and the positive control were prepared in a 96-well plate, and 8 μL of 10 mM test compound was transferred to 12 μL of ACN. 1:3 serial dilution was performed in a DMSO: ACN mixture (v/v: 40:60). HLM was added to assay wells, and then the test compound or a reference inhibitor solution diluted with serum was added to the designated wells on ice. After the 96-well plate and NADPH solution was pre-incubated at 37°C for 5 min, 15 μL of the pre-warmed 8 mM NADPH solution was added to the assay plate to start the reaction. Incubation time: 5 min for 3A4, 10 min for 1A2, 2B6, 2C8, 2C9 and 2D6, and 45 min for 2C19. The reaction was stopped by adding IS-containing ACN.

The sigmoid (non-linear) dose-response model (GraphPad Prism 5.0 or Xlfit model 205) was used to calculate the IC50 by curve fitting, and the data calculation formula was as follows:

$$Y=Bottom + (Top-Bottom)/(1+10^{\wedge}((LogEC50-X)*HillSlope))$$

X is the logarithm of the concentration. Y is the sigmoid response from bottom to top when the concentration goes

from high to low.

Results: Compound EXP-3 had no inhibitory effect.

Embodiment 15: Pharmacokinetic study

For the intravenous route and the oral route, the compound was formulated in an aqueous solution containing 5% DMSO + 95% hydroxypropyl-β-cyclodextrin. Test animals were administered orally at 1-10 mg/kg and intravenously at 1-5 mg/kg. Each group included 3 rats. About 0.2 mL of blood was collected by jugular vein puncture with heparin sodium anticoagulant at the following time points: 0.083 h, 0.25 h, 0.5 h, 1 h, 2 h, 4 h, 6 h, 8 h, 10 h and 24 h. Plasma was separated by centrifugation at 5000-13000 rpm for 10 min within 1 hour after collecting the blood sample, and the plasma sample was analyzed on LC-MS/MS.

Pharmacokinetic parameters

The pharmacokinetic parameters were calculated using Phoenix WinNonlin 7.0 based on blood drug concentration data at different time points to provide parameters such as $AUC_{0-t}$, $AUC_{0-\infty}$, $MRT_{0-\infty}$, $C_{max}$, $T_{max}$, and $T_{1/2}$.

Pharmacokinetic parameters of compound EXP-1:
Oral: AUC is 12043 h*ng/mL, Cmax is 2626 ng/mL, $T_{1/2}$ is 2.68 h.

Pharmacokinetic parameters of filgotinib:
Oral: AUC is 2849 h*ng/mL, Cmax is 426 ng/mL, $T_{1/2}$ is 3.9 h.

Embodiment 16: hERG test

For the purpose of evaluating the risk of prolonged QT interval in ECG of the compound according to the present invention, HEK293 cells expressing humanether-a-go-go related gene (hERG) channels were used to study the effect of the compound according to the present invention on the delayed rectifier K+ current (Ikr) playing an important role in ventricular repolarization.

The cells are maintained at -80 mV using a fully automatic patch clamp system (patch clamp PC-505B (WARNER instruments)) through a fully automatic patch clamp method, and then depolarized to 40 mV with a square wave lasting 4s and hyperpolarized to -40 mV with a square wave lasting 2s to obtain the hERG tail current. The maximum current induced by the second square wave was detected. After this current was stabilized, the test compound was perfused. After the reaction was stabilized, the blocking strength was calculated.

Extracellular fluid (mM): NaCl, 137; KCl, 4; $CaCl_2$, 1.8; $MgCl_2$, 1; HEPES, 10; glucose 10; pH 7.4 (NaOH titration). Intracellular fluid (mM): K Aspartate, 130; $MgCl_2$, 5; EGTA 5; HEPES, 10; Tris-ATP 4; pH 7.2 (KOH titration).

Test result: EXP-3: $IC_{50}$ is greater than 10 μM.

Embodiment 17: Reverse mutation test of *Salmonella typhimurium*

*Salmonella* TA98, TA100, TA1535, TA1537 and *Escherichia coli* were used as test strains (strain source: Molecular Toxicology, Inc.). Ames test was performed under non-metabolic activation conditions and activation conditions to investigate whether the compounds according to the present invention have gene mutation inducing properties.

The cryopreserved strains were resuscitated, inoculated into nutrient broth medium and cultured in a 37°C constant temperature shaker at 100 to 120 rpm for 11 h. The bottom agar medium (containing an appropriate amount of agar, V-B buffer, 20% glucose solution, 20% magnesium sulfate solution) was poured into a dish to cool for later use. 0.1 mL test sample/negative control (DMSO)/positive control solution, 0.1 mL bacterial solution, 0.5 mL of 0.2 mol/L phosphate buffer saline (PBS) (-S9)/0.5 mL S9 mixture (+S9) and 2.5 mL of top medium were respectively added into each test tube, vortexed, mixed well, and then quickly and evenly plated on the bottom agar medium plate. After natural cooling and solidifying, the plate was placed upside down and cultured in a mold incubator at 37°C for 48 h. The cultured plate was removed, counted and compared with the DMSO group for evaluation. If the average number of reverted colonies is twice or more than the number of reverted colonies in the negative control group (which can be regarded as spontaneous reverted number) (TA1535: 3 times or more) and there is a dose-effect relationship, the result is positive, and the test sample can be determined to be a mutagenic agent. Otherwise, the result is negative.

Test result: Compound EXP-3 is negative.

Embodiment 18: Pharmacodynamics model in vivo

18.1 CIA Model

18.1.1 Materials

Complete Freund's adjuvant (CFA) and type II bovine collagen were purchased from Chondrex.

18.1.2 Animals

Lewis rats (female, 10-12 weeks old) were obtained from Beijing Vital River Laboratory Animal Technology Co., Ltd. The rats were on a light and dark alternation for 12 h, and the environment was kept at a temperature of 23±2°C and a humidity of 40-70%.

18.1.3 Collagen-induced arthritis (CIA)

Type II collagen solution (2 mg/mL) was prepared with 0.05 M acetic acid solution to allow to be fully dissolved, and placed overnight in the dark at 4°C. During the test, 2 mg/mL type II collagen (C II) solution and 4 mg/mL complete Freund's adjuvant (CFA) solution were mixed in equal volumes under ice bath conditions to fully emulsify. On day 0, an emulsion prepared by mixing equal volumes of C II (2 mg/mL) and CFA (4 mg/mL) was injected intracutaneously into the base of the tail of experimental rats for primary immunization, and on day 7, secondary booster immunization was performed.

18.1.4 Study design

The therapeutic effect of the test compound was tested in a rat CIA model. The rats were randomly divided into groups with 8 animals in each group. All animals were immunized on day 0 and boosted on day 7. A blank control (Naive group) and a vehicle control group (Vehicle group) were administered with 20% HP-β-CD solution containing DMSO, a positive control group was administered with Filgotinib (10 mg/kg, administered for 2 weeks), tofacitinib citrate (Tofacitinib group, 2 mg/kg, administered for 2 weeks) and upadacitinib (Upadacitinib group, 2 mg/kg, administered for 2 weeks), respectively, and experimental groups were administered with 2 mg/kg, 3 mg/kg, 5mg/kg, and 10 mg/kg of EXP-3 for two weeks, respectively.

18.1.5 Clinical evaluation of arthritis

Foot volume was scored for the swelling of four toes at the same time, the score for each foot was 0-4, and the highest score for each rat was 16. 0-no swelling, normal appearance; 1-mild swelling or redness of the ankle joint, wrist joint or finger joint; 2-moderate swelling or redness of the ankle joint, wrist joint or finger joint; 3-severe swelling or redness of the ankle joint, wrist joint or finger joint; 4-very severe swelling or redness of the ankle joint, wrist joint or finger joint. Pathology score data was analyzed using Mann-Whitney U test as the SPSS non-parametric test, and $p<0.05$ was considered as a significant difference.

18.1.6 Weight and foot volume after onset of arthritis

In clinical practice, weight loss and foot volume reduction were associated with arthritis. Therefore, the differences in body weight and foot volume after the onset of arthritis compared with the negative group, respectively, can be used to evaluate the therapeutic effect in the rat model. The experimental data were expressed as Mean±SEM; the data between the two groups was tested by unpaired t-test and $p<0.05$ was considered as a significant difference.

18.1.7 Test result

18.1.7.1 Effect of test samples on clinical observation and body weight of experimental rats

There were no obvious abnormalities visible to naked eyes during the experimental period of all animals. The body weight of the animals in the Naïve group increased steadily during the experimental period. The body weight of the animals in the Vehicle group was significantly lower than that of the Naïve group on days 8 to 28 after the primary immunization (p<0.01-0.001). The body weight of animals in the Tofacitinib citrate (Tofacitinib) 2 mg/kg and EXP-3 10 mg/kg groups increased slightly on days 11-17, but then fell back. The body weights of the animals in the remaining

administration groups were similar to those in the Vehicle group. Except for the Naive group, there were no statistically significant differences among the remaining groups, which indicated that the various test sample groups had little effect on the body weight of the rats.

**Table 9.** Effect of test samples administered by oral gavage once daily for 2 w on body weight of CIA rats (n=8, $\bar{x} \pm s$)

| Treatment | Body weight (g)/Day | | | | | | | |
|---|---|---|---|---|---|---|---|---|
| | 0 | 8 | 11 | 14 | 17 | 21 | 24 | 28 |
| Naïve | 1.6 | 187.5± 1.8 ** | 193.4± 1.6 ** | 196.8 ± 1.8 *** | 200.8± 2.0 | 204.6± 1.8 | 204.5± 1.5 | 207.1± 1.3 |
| Vehicle | 176.6 ± 1.7 | 174.8± 3.4 | 173.5± 5.4 | 173.6 ± 2.6 | 171.3 ± 1.9 | 171 ± 2.3 | 169.3 ± 2.6 | 174.1 ± 2.1 |
| filgotinib 10 mg/kg | 174.3 ± 2.4 | 171.6± 2.6 | 171.5± 3.6 | 173.3 ± 1.8 | 171.6 ± 1.7 | 165.6 ± 1.6 | 167 ± 1.6 | 169.3 ± 2.1 |
| Tofacitinib 2 mg/kg | 173.8± 2 | 175.4± 2.8 | 176.1± 4.4 | 177 ± 2.7 | 177.1 ± 2.6 | 173.1 ± 2.2 | 171 ± 1.8 | 172.5 ± 1.9 |
| Upadacitinib 2 mg/kg | 172.3 ± 1.8 | 170.4± 2.1 | 169.4± 2.8 | 170 ± 2.2 | 170.8 ± 3.3 | 168.6 ± 2.9 | 165.9 ± 2.9 | 170.5 ± 3.1 |
| EXP-3 10 mg/kg | 173.9 ±1.8 | 175.3 ±2.1 | 175.9 ±2.8 | 175.3 ±2.2 | 176.4 ±3.3 | 171.8 ±2.9 | 166.3 ±2.9 | 170.4 ±3.1 |
| EXP-3 5 mg/kg | 171.8 ±2. 3 | 171.1 ±3 .1 | 170.9 ±4. 5 | 171.4 ±2.4 | 172.8 ±2. 2 | 166.5 ±1.9 | 165.3 ±1.7 | 169.1 ±2.1 |
| EXP-3 3 mg/kg | 174.5 ±1.3 | 172.4 ±2.7 | 170.8 ±3.8 | 171.6 ±2.9 | 170.1 ±2.7 | 166.8 ±1.8 | 165 ±1.8 | 169.3 ±2 |
| EXP-3 2 mg/kg | 172.3 ±1.9 | 172.6 ±0.7 | 172.3 ±1.8 | 170.3±1 | 169.1 ±1.2 | 164.9 ±1.8 | 166.4 ±2.6 | 166.9 ±2.7 |

18.1.7.2 Effect of test samples on hindfoot foot volume of experimental rats

The foot volume of the animals in the Vehicle group was significantly increased on days 14-28 after the primary immunization and began to decrease on day 24. The foot volume in the Upadacitinib 2 mg/kg group was significantly lower on days 17-28 compared to that in the Vehicle group ($p<0.05-0.001$). The remaining administration groups showed a significant decrease in foot volume on days 21-28 compared to the Vehicle group ($p<0.05-0.001$). EXP-3 10mg/kg was the most potent and significantly stronger than filgotinib 10 mg/kg, followed by Upadacitinib 2 mg/kg. EXP-3 5 mg/kg-2 mg/kg was similar to Tofacitinib citrate (Tofacitinib) 2 mg/kg and slightly weaker than Upadacitinib 2 mg/kg. (Table 10). It indicates that the test samples have certain improvement effect on the foot volume of rats in CIA model.

**Table 10.** Effect of test samples administered by oral gavage once daily for 2 w on hindfoot foot volume of CIA rats (n=8, $\bar{x}\pm s$)

| Treatment | Foot volume (ml)/Day | | | | | | | |
|---|---|---|---|---|---|---|---|---|
| | 0 | 8 | 11 | 14 | 17 | 21 | 24 | 28 |
| Naïve | 2.684±0.012 | 2.685±0.012 | 2.694±0.017 | 2.702±0.012 ** | 2.734±0.021 *** | 2.72±0.027 *** | 2.746±0.034 *** | 2.735±0.035 *** |
| Vehicle | 2.619±0.033 | 2.621±0.027 | 2.742±0.046 | 3.849±0.320 | 5.058±0.254 | 5.535±0.122 | 5.379±0.140 | 4.934±0.144 |
| Filgotinib 10 mg/kg | 2.597±0.014 | 2.589±0.020 | 2.700±0.090 | 3.644±0.330 | 4.702±0.255 | 4.829±0.153 ** | 4.769±0.093 ** | 4.363±0.130 * |
| Tofacitinib 2 mg/kg | 2.637±0.020 | 2.619±0.019 | 2.646±0.045 | 3.848±0.333 | 4.636±0.306 | 4.710±0.061 *** | 4.509±0.092 *** | 4.125±0.094 *** |
| Upadacitinib 2 mg/kg | 2.611±0.022 | 2.599±0.020 | 2.738±0.110 | 3.675±0.314 | 4.126±0.301 * | 4.591±0.163 *** | 4.403±0.167 *** | 3.990±0.106 *** |
| EXP-3 10 mg/kg | 2.645±0.017 | 2.613±0.017 | 2.647±0.075 | 3.652±0.287 | 4.249±0.342 | 4.331±0.097 *** | 4.125±0.075 *** *** | 3.826±0.075 *** |
| EXP-3 5 mg/kg | 2.631±0.017 | 2.611±0.017 | 2.703±0.061 | 3.684±0.315 | 4.516±0.302 | 4.724±0.121 *** | 4.582±0.099 *** | 4.176±0.065 *** |
| EXP-3 3 mg/kg | 2.633±0.017 | 2.624±0.010 | 2.706±0.037 | 3.724±0.239 | 4.759±0.291 | 4.863±0.080 *** | 4.754±0.086 ** | 4.299±0.082 ** |
| EXP-3 2 mg/kg | 2.603±0.021 | 2.590±0.027 | 2.634±0.059 | 3.648±0.303 | 4.465±0.261 | 4.600±0.178 *** | 4.487±0.174 ** | 4.181±0.152 ** |

*$p<0.05$, **$p<0.01$, ***$p<0.001$ vs. Vehicle

18.1.7.3 Effect of test samples on arthritis index (AI) of extremities of experimental rats

The experimental animals began to develop swelling of the extremities on days 9-10 after the primary immunization, most of the feet being attacked reached 2-3 points on day 14, and the drug administration was started for each group. The results showed that the AI scores of the animals in the Vehicle group continued to increase on days 11-24 and decreased slightly on day 28. EXP-3 10 mg/kg had the strongest effect on reducing swelling of the extremities, followed by tofacitinib 2 mg/kg. EXP-3 5 mg/kg, EXP-3 2 mg/kg and Upadacitinib 2 mg/kg had similar effects, which were slightly weaker than tofacitinib 2 mg/kg. Filgotinib was the least potent. (Table 11). It indicates that the test samples have certain improvement effect on the arthritic condition of the extremities of rats in CIA model.

**Table 11.** Effect of test samples administered by oral gavage once daily for 2 w on arthritis index of CIA rats (n=8, $\bar{x}\pm s$)

| Treatment | Arthritis index/Day | | | | | | | |
|---|---|---|---|---|---|---|---|---|
| | 0 | 8 | 11 | 14 | 17 | 21 | 24 | 28 |
| Naïve | 0±0 | 0±0 | 0 ±0* | 0±0*** | 0±0*** | 0±0*** | 0±0*** | 0±0*** |
| Vehicle | 0±0 | 0±0 | 0.88 ±0.30 | 3.88 ±1.04 | 7.88 ±1.38 | 11.88 ±1.20 | 12.75 ±0.96 | 11.88 ±0.48 |
| filgotinib 10 mg/kg | 0 ±0 | 0±0 | 0.75 ±0.41 | 3.88 ±0.83 | 6.50 ±1.05 | 10.00 ±1.07 | 11.25 ±0.73 | 10.00 ±0.46* |
| tofacitinib 2 mg/kg | 0 ±0 | 0±0 | 0.63 ±0.32 | 3.75 ±0.77 | 5.25 ±0.62 | 8.75 ±0.59 | 8.75 ±0.73* | 7.88 ±0.58*** |
| Upadacitinib 2 mg/kg | 0 ±0 | 0±0 | 0.63 ±0.38 | 3.50 ±0.73 | 5.50 ±1.00 | 9.00 ±1.07 | 9.38 ±0.71* | 8.75 ±0.56** |
| 10 mg/kg | 0 ±0 | 0±0 | ±0.38 | ±0.70 | 5.00 ±0.89 | ±0.50** | ±0.56** | ±0.41*** |
| EXP-3 5 mg/kg | 0 ±0 | 0±0 | 0.63 ±0.26 | 3.50 ±0.76 | 5.88 ±1.22 | 9.00 ±1.22 | 9.13 ±1.04* | 8.00 ±0.65*** |
| EXP-3 3 mg/kg | 0 ±0 | 0±0 | 0.38 ±0.18 | 3.75 ±0.70 | 6.25 ±0.75 | 10.25 ±0.70 | 10.88 ±0.44 | 9.25 ±0.45** |
| EXP-3 2 mg/kg | 0 ±0 | 0±0 | 0.50 ±0.33 | 3.63 ±0.91 | 6.25 ±1.36 | 9.00 ±1.24 | 9.13 ±1.04* | 8.63 ±0.63** |

*$p$<0.05, **$p$<0.01, ***$p$<0.001 vs. Vehicle

## Claims

1. A compound represented by formula (I) or a stereoisomer or tautomer thereof or a pharmaceutically acceptable salt thereof or a solvate or prodrug thereof

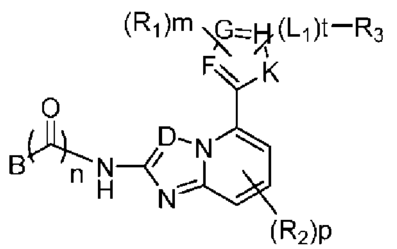

Formula I

wherein:

B is independently selected from substituted or unsubstituted $C_3$-$C_6$ cycloalkyl, aryl, and 5- to 6-membered

heteroaryl ring having 1 to 2 ring heteroatoms independently selected from N, O and S, wherein the $C_3$-$C_6$ cycloalkyl or 5- to 6-membered heteroaryl ring is optionally substituted with one or more substituents independently selected from halogen, fluorinated or unfluorinated $C_1$-$C_6$ alkyl, fluorinated or unfluorinated $C_1$-$C_6$ alkoxy, and fluorinated or unfluorinated $C_1$-$C_6$ alkylamino;
D is independently selected from C and N;
F, G, H, and K are independently selected from C, N, S, and O;
$L_1$ is absent or is independently selected from single bond, $-CH_2-$, $-(CH_2)_xO(CH_2)_y-$, $-(CH_2)_xNH(CH_2)_y-$, $-CH_2O-$, -C(O)-, $-CON(R_4)-$, $-CH_2N(R_4)-$, $-CONH(CH_2)_y-$, $-N(R_4)-$, $-SO_2N(R_4)-$, $-S(O)_2-$, and -N(Me)-;
$R_4$ is independently selected from H, $C_1$-$C_6$ alkyl, substituted $C_1$-$C_6$ alkyl, $C_1$-$C_3$ ether $C_1$-$C_3$ alkyl, and methylsulfonyl;
$R_1$ is independently selected from H, $-NH_2$, -COOH, substituted or unsubstituted $C_1$-$C_6$ alkyl, acyl, substituted or unsubstituted acylamino, substituted or unsubstituted $C_1$-$C_6$ alkoxy, halogen, hydroxyl, substituted or unsubstituted $C_1$-$C_3$ ester group, and substituted or unsubstituted heteroaryl;
$R_2$ is absent or is independently selected from H, F, Cl, and Me;
$R_3$ is independently selected from H, substituted or unsubstituted sulfonyl, substituted or unsubstituted sulfonamido, substituted or unsubstituted 5- to 6-membered heterocyclic ring having at least one heteroatom and optionally having a second ring heteroatom independently selected from N and S, substituted or unsubstituted $C_1$-$C_6$ alkane, and a substituent is independently selected from 5- to 6-membered heteroaryl and methoxy, substituted or unsubstituted $C_3$-$C_7$ cycloalkyl, substituted or unsubstituted 4- to 7-membered heterocycloalkyl, and substituted or unsubstituted 5- to 7-membered heteroaryl; and
m is 0, 1, 2, or 3; n is 0, 1, 2, or 3; p is 0, 1, or 2; and t is 0, 1, 2, or 3.

**2.** The compound represented by formula (I) or a stereoisomer or tautomer thereof or a pharmaceutically acceptable salt thereof or a solvate or prodrug thereof according to claim 1, wherein B is selected from the following substituted or unsubstituted groups: cyclopropane, cyclobutane, pyrazolyl, pyridyl, and imidazolyl; and a substituent is selected from F, Cl, Br, methyl, ethyl, propyl, isopropyl, and trifluoromethyl.

**3.** The compound represented by formula (I) or a stereoisomer or tautomer thereof or a pharmaceutically acceptable salt thereof or a solvate or prodrug thereof according to claim 1, wherein F, G, H, and K are not C at the same time; preferably, K is S, and F, G, and H are C.

**4.** The compound represented by formula (I) or a stereoisomer or tautomer thereof or a pharmaceutically acceptable salt thereof or a solvate or prodrug thereof according to claim 1, wherein D is C or N; n is 1; m is 0 or 1; preferably, m is 0; $R_2$ is absent or is H; p is 0; and t is 1.

**5.** The compound represented by formula (I) or a stereoisomer or tautomer thereof or a pharmaceutically acceptable salt thereof or a solvate or prodrug thereof according to claim 1, wherein $R_1$ is independently selected from H, $-NH_2$, -COOH, hydroxyl, halogen, substituted or unsubstituted $C_1$-$C_3$ alkyl, substituted or unsubstituted $C_1$-$C_3$ acyl, substituted or unsubstituted $C_1$-$C_3$ acylamino, substituted or unsubstituted $C_1$-$C_3$ alkoxy, and substituted or unsubstituted $C_1$-$C_3$ ester group; preferably, $R_1$ is independently selected from H, $-NH_2$, -COOH, hydroxyl, halogen, methyl, ethyl, propyl, isopropyl, formylamino and carbomethoxy; $L_1$ is absent or independently selected from single bond, $-CH_2-$, $-(CH_2)_xO(CH_2)_y-$, $-(CH_2)_xNH(CH_2)_y-$, $-CONH(CH_2)_y-$, and $-N(R_4)-$; $R_4$ is independently selected from H, methyl, ethyl, propyl, ethyl methyl ether, methyl methyl ether, ethyl ethyl ether; x or y is independently selected from 0, 1 or 2; preferably, $L_1$ is absent or is independently selected from single bond, $-CH_2-$, $-(CH_2)_xO(CH_2)_y-$, $-(CH_2)_xNH(CH_2)_y-$, $-CONH(CH_2)_y-$, and $-N(R_4)-$; $R_4$ is independently selected from H, methyl, and ethyl methyl ether; and x or y is independently selected from 0, 1 or 2.

**6.** The compound represented by formula (I) or a stereoisomer or tautomer thereof or a pharmaceutically acceptable salt thereof or a solvate or prodrug thereof according to claim 1, wherein $R_3$ is independently selected from H and the following substituted or unsubstituted groups: sulfonyl, sulfonamido, thiomorpholine 1,1-dioxide, piperidine, pyrazole, thiazole, imidazole, 1,3,4-thiadiazole, piperazine, morpholine, thiophene, oxazole, 1,3,4-oxadiazole, furan, pyrrole, 3-pyrroline, 2-pyrazoline, 1,2,3-azole, 1,2,3-triazole, 1,2,4-triazole, and pyran; and a substituent is selected from H, halogen, $C_1$-$C_3$ alkyl, $C_1$-$C_3$ haloalkyl, hydroxy $C_1$-$C_3$ alkyl, $C_3$-$C_6$ cycloalkyl, ethyl methyl ether, methyl methyl ether, and ethyl ethyl ether; preferably, $R_3$ is independently selected from H and the following substituted or unsubstituted groups: sulfonyl, sulfonamido, thiomorpholine 1,1-dioxide, piperidine, pyrazole, thiazole, imidazole, 1,3,4-thiadiazole, piperazine, morpholine, pyrazine, thiophene, oxazole, 1,3,4-oxadiazole, furan, pyrrole, 3-pyrroline, 2-pyrazoline, 1,2,3-azole, 1,2,3-triazole, 1,2,4-triazole, and pyran; and a substituent is selected from H, halogen, methyl, ethyl, propyl, isopropyl, trifluoromethyl, hydroxyethyl, hydroxymethyl, cyclopropane, cyclobutane, ethyl methyl ether, methyl methyl ether, and ethyl ethyl ether.

**7.** The compound represented by formula (I) or a stereoisomer or tautomer thereof or a pharmaceutically acceptable salt thereof or a solvate or prodrug thereof according to claim 1, wherein $R_3$ is independently selected from H and the following substituted or unsubstituted groups: sulfonyl, sulfonamido, thiomorpholine 1,1-dioxide, piperidine, pyrazole, thiazole, imidazole, 1,3,4-thiadiazole, piperazine, morpholine, thiophene, oxazole, 1,3,4-oxadiazole, furan, pyrrole, 3-pyrroline, 2-pyrazoline, 1,2,3-azole, 1,2,3-triazole, 1,2,4-triazole, and pyran; and a substituent is selected from $C_1$-$C_3$ haloalkoxy; preferably, $R_3$ is independently selected from H and the following substituted or unsubstituted groups: sulfonyl, sulfonamido, thiomorpholine 1,1-dioxide, piperidine, pyrazole, thiazole, imidazole, 1,3,4-thiadiazole, piperazine, morpholine, pyrazine, thiophene, oxazole, 1,3,4-oxadiazole, furan, pyrrole, 3-pyrroline, 2-pyrazoline, 1,2,3-azole, 1,2,3-triazole, 1,2,4-triazole, and pyran; and the substituent is selected from trifluoromethoxy.

**8.** A compound represented by the following structure, or a stereoisomer or tautomer thereof, or a pharmaceutically acceptable salt thereof, or a solvate or prodrug thereof:

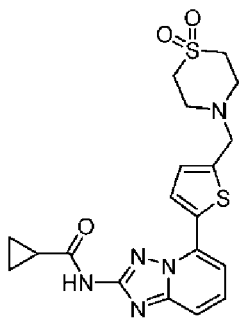

**EXP-1**

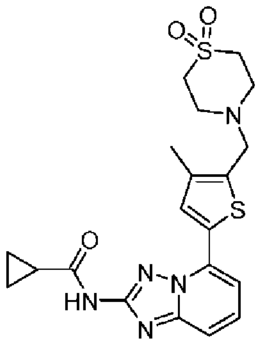

EXP-2

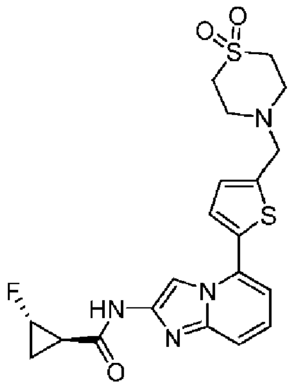

EXP-3

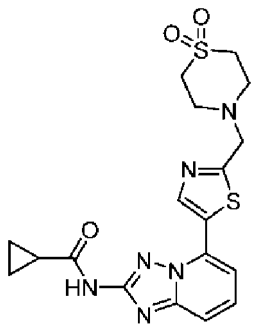

**EXP-4**

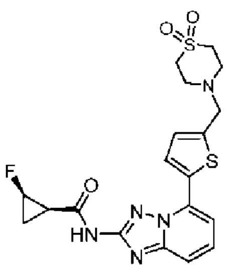

**EXP-5**

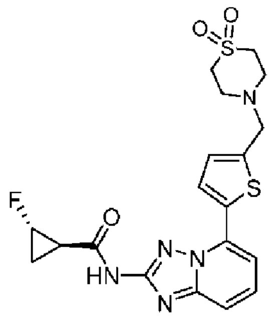

EXP-6

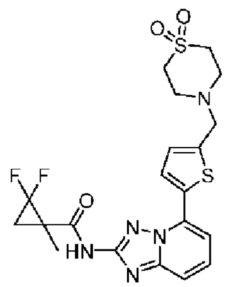

EXP-7

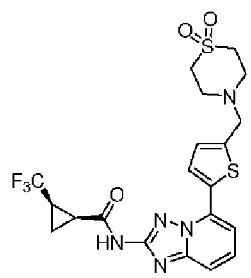

EXP-8

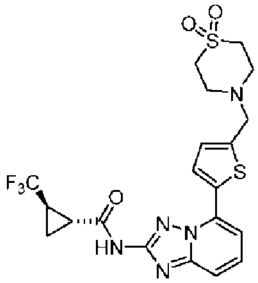

EXP-9

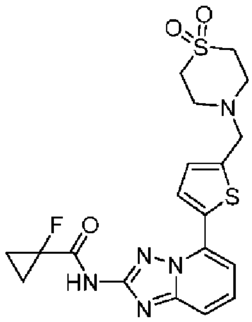

EXP-10

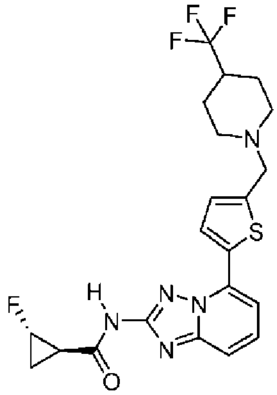

EXP-11

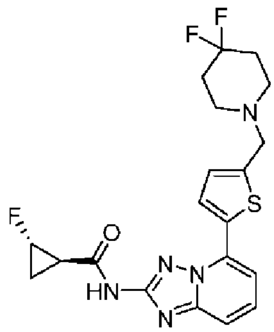

EXP-12

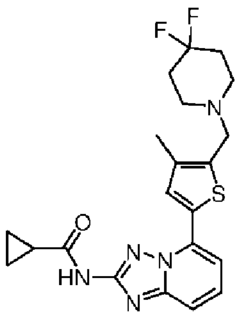

EXP-13

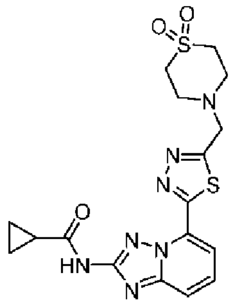

**EXP-14**

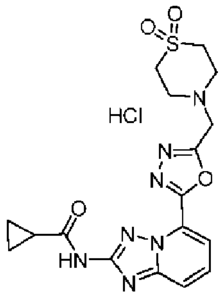

**EXP-15**

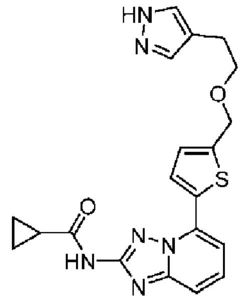

**EXP-16**

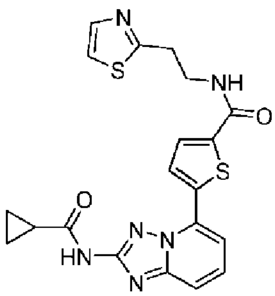

**EXP-17**

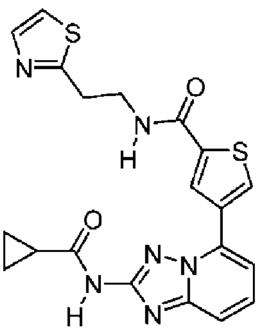

EXP-18

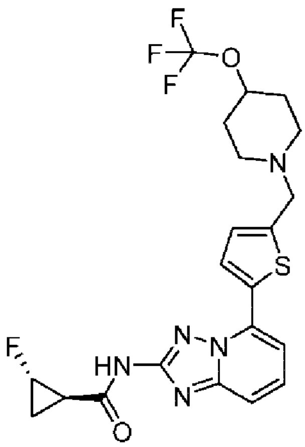

**EXP-19**

.

9. A pharmaceutical composition comprising the compound according to any of claims 1-7, or a stereoisomer or tautomer thereof or a pharmaceutically acceptable salt thereof, and a pharmaceutically acceptable carrier.

10. Use of the compound according to any of claims 1-6 in preparation of drugs for preventing or treating JAK kinase-related diseases, preferably, in preparation of drugs for preventing and/or treating diseases involving cartilage degeneration and bone and/or joint degeneration, conditions involving inflammation or immune response, endotoxin-driven disease states, cancer, and organ transplantation rejection; and more preferably, in preparation of drugs for preventing and/or treating osteoarthritis, Crohn’s disease, rheumatoid arthritis, psoriasis, allergic airway disease, juvenile idiopathic arthritis, colitis, inflammatory bowel disease, endotoxin-driven disease state, disease with cartilage renewal damage, congenital cartilage deformity, organ transplantation rejection, cartilage degeneration, joint degeneration, myeloproliferative disorder, leukemia, and solid tumor.

**INTERNATIONAL SEARCH REPORT**

International application No.
**PCT/CN2020/107028**

**A. CLASSIFICATION OF SUBJECT MATTER**

C07D 471/04(2006.01)i; A61P 19/08(2006.01)i; A61P 19/02(2006.01)i; A61P 17/06(2006.01)i; A61P 11/00(2006.01)i; A61P 35/00(2006.01)i; A61P 35/02(2006.01)i; A61P 19/00(2006.01)i; A61P 37/06(2006.01)i; A61P 37/08(2006.01)i; A61K 31/437(2006.01)i; A61K 31/4545(2006.01)i; A61K 31/54(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

Database: CNABS, VEN, CNKI, CNTXT, EPTXT, WOTXT, USTXT, 万方, WANFANG, 百度, BAIDU, Registry, Caplus; Keywords: JAK激酶, 抑制剂, 三唑并[1, 5-a]吡啶, 三氮唑并[1, 5-a]吡啶, 环丙烷, 酰胺, 噻吩, 硫代吗啉; JAK, inhibitor, triazolo[1, 5-a]pyridin, cycloprop+, amino, thiophen, thiomorpholin+.

**C. DOCUMENTS CONSIDERED TO BE RELEVANT**

| Category* | Citation of document, with indication, where appropriate, of the relevant passages | Relevant to claim No. |
|---|---|---|
| X | CN 102105471 A (GALAPAGOS N. V.) 22 June 2011 (2011-06-22)<br>description, pages 4-17, 19, 111 and 130-132 | 1-10 |
| Y | CN 102105471 A (GALAPAGOS N. V.) 22 June 2011 (2011-06-22)<br>description, pages 4-17, 19, 111 and 130-132 | 1-3, 5-7, 9, 10 |
| X | CN 102131390 A (GENENTECH INC.) 20 July 2011 (2011-07-20)<br>description, pages 13, 14, 49-53, 72, 82, 83 and 118 | 1-3, 5-7, 9, 10 |
| X | CN 102203093 A (SIGNAL PHARM LLC) 28 September 2011 (2011-09-28)<br>description, pages 4-6, 11, 50-55, 296, 362 and 370 | 1-3, 5-7, 9, 10 |
| X | WO 2009047514 A1 (CANCER RESEARCH TECHNOLOGY LIMITED) 16 April 2009 (2009-04-16)<br>pages 13, 62-64, 73, 77, 79, 81, 82 and 219-225 | 1-7, 9, 10 |
| X | CN 101878212 A (SERONO LAB) 03 November 2010 (2010-11-03)<br>description, pages 31, 52, 54-57, 59-61, 63-65, 70, 71, 90 and 91 | 1-7, 9, 10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 September 2020** | **12 November 2020** |

| Name and mailing address of the ISA/CN | Authorized officer |
|---|---|
| **China National Intellectual Property Administration (ISA/CN)<br>No. 6, Xitucheng Road, Jimenqiao Haidian District, Beijing 100088<br>China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/107028**

**C. DOCUMENTS CONSIDERED TO BE RELEVANT**

| Category* | Citation of document, with indication, where appropriate, of the relevant passages | Relevant to claim No. |
|---|---|---|
| X | CN 107759587 A (CHINA MEDICINE RESEARCH & DEVELOPMENT CENTER CO., LTD.) 06 March 2018 (2018-03-06)<br>description, pages 3-5 and 9-11 | 1-10 |
| Y | CN 107759587 A (CHINA MEDICINE RESEARCH & DEVELOPMENT CENTER CO., LTD.) 06 March 2018 (2018-03-06)<br>description, pages 3-5 and 9-11 | 1-3, 5-7, 9, 10 |
| X | CN 101535307 A (CELLZOME UK. LTD.) 16 September 2009 (2009-09-16)<br>description, pages 13 and 19-29 | 1-7, 9, 10 |
| X | WO 2010010188 A1 (GALAPAGOS N. V.) 28 January 2010 (2010-01-28)<br>pp. 26-36 | 1-3, 5-7, 9, 10 |
| X | Christel J. Menet et al. "Triazolopyridines as Selective JAK1 Inhibitors: From Hit Identification to GLPG0634"<br>*Journal of Medicinal Chemistry,* Vol. 57, No. 22, 04 November 2014 (2014-11-04), ISSN: 0022-2623,<br>pp. 9323-9342 | 1-7, 9, 10 |
| X | Michael Siu et al. "2-Amino-[1, 2, 4]triazolo[1, 5-a]pyridines as JAK2 inhibitors"<br>*Bioorganic & Medicinal Chemistry Letters,* Vol. 23, No. 17, 15 June 2013 (2013-06-15), ISSN: 0960-894X,<br>pp. 5014-5021 | 1-3, 5-7, 9, 10 |
| A | CN 108341814 A (SHANGHAI XIANGJIN BIOTECHNOLOGY CO., LTD.) 31 July 2018 (2018-07-31)<br>entire document | 1-10 |
| A | WO 2017133423 A1 (SHENZHEN TARGETRX, INC.) 10 August 2017 (2017-08-10)<br>entire document | 1-10 |
| A | WO 2016179207 A1 (CONCERT PHARMACEUTICALS, INC.) 10 November 2016 (2016-11-10)<br>entire document | 1-10 |

**INTERNATIONAL SEARCH REPORT**
**Information on patent family members**

International application No.
**PCT/CN2020/107028**

| Patent document cited in search report | Publication date (day/month/year) | Patent family member(s) | Publication date (day/month/year) |
|---|---|---|---|
| CN 102105471 A | 22 June 2011 | PE 20100152 A1 | 24 March 2010 |
| | | CA 2730762 C | 21 November 2017 |
| | | MX 2011000756 A | 21 March 2011 |
| | | IL 210262 D0 | 31 March 2011 |
| | | HR P20130357 T1 | 31 May 2013 |
| | | KR 101711911 B1 | 03 March 2017 |
| | | CY 1113945 T1 | 27 July 2016 |
| | | PL 2361251 T3 | 31 October 2014 |
| | | CA 2730757 A1 | 28 January 2010 |
| | | ES 2406691 T3 | 07 June 2013 |
| | | DK 2346864 T3 | 13 May 2013 |
| | | EP 2361251 A1 | 31 August 2011 |
| | | RS 52823 B | 31 October 2013 |
| | | SI 2346864 T1 | 31 May 2013 |
| | | JO 3041 B1 | 05 September 2016 |
| | | WO 2010010190 A1 | 28 January 2010 |
| | | UY 32006 A | 26 February 2010 |
| | | IL 210261 A | 30 March 2017 |
| | | CN 102105471 B | 15 October 2014 |
| | | ME 02046 B | 20 May 2015 |
| | | SI EP2361251 T1 | 30 September 2014 |
| | | CL 2009001637 A1 | 19 February 2010 |
| | | KR 20110045020 A | 03 May 2011 |
| | | PT 2361251 E | 11 July 2014 |
| | | CA 2730762 A1 | 28 January 2010 |
| | | JP 2011529033 A | 01 December 2011 |
| | | EA 201170255 A1 | 30 August 2011 |
| | | JP 5559168 B2 | 23 July 2014 |
| | | WO 2010010191 A1 | 28 January 2010 |
| | | EP 2346864 A1 | 27 July 2011 |
| | | KR 101676391 B1 | 15 November 2016 |
| | | US 2010029709 A1 | 04 February 2010 |
| | | EA 018587 B1 | 30 September 2013 |
| | | US 2012309784 A1 | 06 December 2012 |
| | | EA 018080 B1 | 30 May 2013 |
| | | HK 1158634 A1 | 08 November 2013 |
| | | US 8242274 B2 | 14 August 2012 |
| | | NZ 590646 A | 25 November 2011 |
| | | EA 201170257 A1 | 30 August 2011 |
| | | ES 2485913 T3 | 14 August 2014 |
| | | US 2015150856 A1 | 04 June 2015 |
| | | HK 1158635 A1 | 05 December 2014 |
| | | BR PI0916862 A2 | 10 February 2016 |
| | | US 8853240 B2 | 07 October 2014 |
| | | AU 2009273144 A1 | 28 January 2010 |
| | | KR 20110053435 A | 23 May 2011 |
| | | TW 201008939 A | 01 March 2010 |
| | | EP 2361251 B1 | 30 April 2014 |
| | | MY 157615 A | 30 June 2016 |
| | | US 2017035738 A1 | 09 February 2017 |

Form PCT/ISA/210 (patent family annex) (January 2015)

**INTERNATIONAL SEARCH REPORT**
**Information on patent family members**

International application No.
**PCT/CN2020/107028**

| Patent document cited in search report | Publication date (day/month/year) | Patent family member(s) | Publication date (day/month/year) |
|---|---|---|---|
| | | US 2011190260 A1 | 04 August 2011 |
| | | US 10206907 B2 | 19 February 2019 |
| | | US 9415037 B2 | 16 August 2016 |
| CN 102131390 A | 20 July 2011 | CL 2010001486 A1 | 06 May 2011 |
| | | AU 2009259867 A1 | 23 December 2009 |
| | | JP 5595389 B2 | 24 September 2014 |
| | | WO 2009155565 A1 | 23 December 2009 |
| | | CA 2726844 A1 | 23 December 2009 |
| | | US 2014378449 A1 | 25 December 2014 |
| | | RU 2561104 C2 | 20 August 2015 |
| | | JP 2011525193 A | 15 September 2011 |
| | | CA 2726844 C | 30 August 2016 |
| | | KR 20110031475 A | 28 March 2011 |
| | | EP 2296475 A4 | 05 March 2014 |
| | | SG 178807 A1 | 29 March 2012 |
| | | US 2016024089 A1 | 28 January 2016 |
| | | BR PI0909945 A2 | 28 July 2015 |
| | | RU 2011101901 A | 27 July 2012 |
| | | PE 20110063 A1 | 16 February 2011 |
| | | IL 209764 D0 | 28 February 2011 |
| | | US 2010035875 A1 | 11 February 2010 |
| | | MX 336271 B | 13 January 2016 |
| | | US 8889673 B2 | 18 November 2014 |
| | | EP 2296475 A1 | 23 March 2011 |
| | | MX 2010014002 A | 15 February 2011 |
| | | US 2012264747 A1 | 18 October 2012 |
| CN 102203093 A | 28 September 2011 | JP 2012502029 A | 26 January 2012 |
| | | CR 20110132 A | 20 June 2011 |
| | | NZ 591551 A | 28 October 2011 |
| | | CA 2735806 A1 | 11 March 2010 |
| | | TW I453207 B | 21 September 2014 |
| | | US 2013005707 A1 | 03 January 2013 |
| | | NI 201100048 A | 17 November 2011 |
| | | CN 104910148 A | 16 September 2015 |
| | | MX 2011002536 A | 04 April 2011 |
| | | AU 2009288618 B2 | 02 October 2014 |
| | | EP 2344494 B1 | 20 April 2016 |
| | | IL 211549 D0 | 31 May 2011 |
| | | CN 104910148 B | 27 June 2017 |
| | | EC SP11010945 A | 31 May 2011 |
| | | CN 102203093 B | 13 May 2015 |
| | | CO 6531502 A2 | 28 September 2012 |
| | | RU 2552642 C2 | 10 June 2015 |
| | | PE 20110387 A1 | 22 June 2011 |
| | | US 2010093698 A1 | 15 April 2010 |
| | | AU 2009288618 A1 | 11 March 2010 |
| | | RU 2011113540 A | 20 October 2012 |
| | | JP 5689060 B2 | 25 March 2015 |
| | | EP 2344494 A1 | 20 July 2011 |
| | | TW 201024293 A | 01 July 2010 |

Form PCT/ISA/210 (patent family annex) (January 2015)

**INTERNATIONAL SEARCH REPORT**
**Information on patent family members**

International application No.
**PCT/CN2020/107028**

| Patent document cited in search report | Publication date (day/month/year) | Patent family member(s) | Publication date (day/month/year) |
|---|---|---|---|
| | | ZA 201101586 B | 30 May 2012 |
| | | BR PI0918854 A2 | 26 February 2019 |
| | | KR 20110053266 A | 19 May 2011 |
| | | IL 211549 A | 30 April 2017 |
| | | US 8299056 B2 | 30 October 2012 |
| | | ES 2581565 T3 | 06 September 2016 |
| | | CL 2011000480 A1 | 16 September 2011 |
| | | WO 2010027500 A1 | 11 March 2010 |
| WO 2009047514 A1 | 16 April 2009 | US 9012633 B2 | 21 April 2015 |
| | | SI EP2217578 T1 | 31 March 2014 |
| | | PL 2217578 T3 | 30 May 2014 |
| | | EP 2217578 A1 | 18 August 2010 |
| | | DK 2217578 T5 | 10 November 2014 |
| | | US 8431596 B2 | 30 April 2013 |
| | | GB 0719803 D0 | 21 November 2007 |
| | | US 2015246915 A1 | 03 September 2015 |
| | | PT 2217578 E | 17 February 2014 |
| | | DK 2217578 T3 | 03 February 2014 |
| | | ES 2446298 T9 | 29 September 2014 |
| | | EP 2217578 B9 | 10 September 2014 |
| | | US 2017066762 A1 | 09 March 2017 |
| | | US 2010298339 A1 | 25 November 2010 |
| | | SI 2217578 T1 | 31 March 2014 |
| | | HR P20140191 T1 | 25 April 2014 |
| | | CY 1114911 T1 | 14 December 2016 |
| | | EP 2217578 B1 | 04 December 2013 |
| | | US 9394301 B2 | 19 July 2016 |
| | | ES 2446298 T3 | 07 March 2014 |
| | | US 2014155594 A1 | 05 June 2014 |
| | | US 9771362 B2 | 26 September 2017 |
| CN 101878212 A | 03 November 2010 | ZA 200909152 B | 30 March 2011 |
| | | US 2012238565 A1 | 20 September 2012 |
| | | AU 2008291075 A8 | 11 March 2010 |
| | | JP 2010536917 A | 02 December 2010 |
| | | US 2010197681 A1 | 05 August 2010 |
| | | CA 2691448 A1 | 05 March 2009 |
| | | KR 20100075881 A | 05 July 2010 |
| | | US 8263595 B2 | 11 September 2012 |
| | | WO 2009027283 A1 | 05 March 2009 |
| | | EA 201070328 A1 | 30 August 2010 |
| | | AU 2008291075 A1 | 05 March 2009 |
| | | EP 2181112 A1 | 05 May 2010 |
| | | MX 2010002312 A | 18 March 2010 |
| CN 107759587 A | 06 March 2018 | None | |
| CN 101535307 A | 16 September 2009 | EP 1894931 A1 | 05 March 2008 |
| | | CN 101535307 B | 30 October 2013 |
| | | JP 2010501633 A | 21 January 2010 |
| | | WO 2008025821 A1 | 06 March 2008 |
| | | US 2010227800 A1 | 09 September 2010 |
| | | EP 2057158 A1 | 13 May 2009 |

Form PCT/ISA/210 (patent family annex) (January 2015)

**INTERNATIONAL SEARCH REPORT**
**Information on patent family members**

International application No.
**PCT/CN2020/107028**

| Patent document cited in search report | Publication date (day/month/year) | Patent family member(s) | Publication date (day/month/year) |
|---|---|---|---|
| | | CN 103641829 A | 19 March 2014 |
| | | JP 5336375 B2 | 06 November 2013 |
| | | US 8883820 B2 | 11 November 2014 |
| | | EP 2057158 B1 | 12 August 2015 |
| | | CA 2662074 A1 | 06 March 2008 |
| | | AU 2007291190 A1 | 19 March 2009 |
| WO 2010010188 A1 | 28 January 2010 | None | |
| CN 108341814 A | 31 July 2018 | None | |
| WO 2017133423 A1 | 10 August 2017 | CN 108349974 A | 31 July 2018 |
| WO 2016179207 A1 | 10 November 2016 | AU 2016257892 A1 | 09 November 2017 |

Form PCT/ISA/210 (patent family annex) (January 2015)